(19) Europäisches Patentamt
European Patent Office
Office européen des brevets

(11) **EP 4 067 903 A1**

(12) **EUROPEAN PATENT APPLICATION**
published in accordance with Art. 153(4) EPC

(43) Date of publication:
**05.10.2022 Bulletin 2022/40**

(21) Application number: **20891661.9**

(22) Date of filing: **18.11.2020**

(51) International Patent Classification (IPC):
***G01N 33/497*** (2006.01)    ***G01N 33/50*** (2006.01)

(52) Cooperative Patent Classification (CPC):
**G01N 33/497; G01N 33/50**

(86) International application number:
**PCT/JP2020/043062**

(87) International publication number:
**WO 2021/106724 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.11.2019 JP 2019213551**

(71) Applicant: **Kyocera Corporation
Kyoto-shi Kyoto 612-8501 (JP)**

(72) Inventor: **ABE Shinichi
Kyoto-shi, Kyoto 612-8501 (JP)**

(74) Representative: **TBK
Bavariaring 4-6
80336 München (DE)**

(54) **STRESS MEASUREMENT SYSTEM AND STRESS MEASUREMENT METHOD**

(57)    Provided is a stress measurement system and a stress measurement method that are capable of measuring the stress level of a subject without taking time and effort. A stress measurement system 1 includes a sensor unit 31 that detects a plurality of detection target gases based on substances contained in a specimen of a subject and outputs a plurality of detection values corresponding to respective detection results of the plurality of detection target gases, and a control unit that determines a stress level of the subject, based on a combination of the plurality of detection values. In addition, the substances contained in the specimen may include a substance serving as a raw material for a brain neurotransmitter.

FIG. 2

EP 4 067 903 A1

**Description**

Cross Reference to Related Applications

The present application claims priority to Japanese Patent Application No. 2019-213551 (filed November 26, 2019), the entire disclosure of which is incorporated herein by reference.

Technical Field

The present disclosure relates to a stress measurement system and a stress measurement method.

Background Art

Examples of known methods for measuring the stress state of a subject include evaluation based on cortisol of blood or the like, and evaluation based on a change in exhalation or heartbeat rate. As another method, a stress measurement device that determines the stress of speakers during a conversation by using voice data of both speakers is proposed (for example, PTL 1 below).

Citation List

Patent Literature

PTL 1: Japanese Unexamined Patent Application Publication No. 2010-273700

Summary of Invention

A stress measurement system according to an embodiment of the present disclosure includes a sensor unit that detects a plurality of detection target gases based on substances contained in a specimen of a subject and outputs a plurality of detection values corresponding to respective detection results of the plurality of detection target gases; and a control unit that determines a stress level of the subject, based on a combination of the plurality of detection values.
Further, a stress measurement method according to an embodiment of the present disclosure includes detecting a plurality of detection target gases from a specimen of a subject and respective concentrations of the plurality of detection target gases; and determining a stress level of the subject, based on a combination of the concentrations of the plurality of detection target gases.

Brief Description of Drawings

[Fig. 1] Fig. 1 is an external view of a stress measurement system according to an embodiment of the present disclosure.
[Fig. 2] Fig. 2 is a diagram illustrating an example internal configuration of a housing included in the stress measurement system.
[Fig. 3] Fig. 3 is a functional block diagram illustrating an example of the stress measurement system.
[Fig. 4] Fig. 4 is a circuit diagram illustrating an example of a sensor unit.
[Fig. 5] Fig. 5 is a diagram illustrating an example of voltage waveforms of sensor units.
[Fig. 6] Fig. 6 is a diagram exemplarily illustrating the relationship between detection values of the concentrations of phenols and indoles and stress of a subject.
[Fig. 7] Fig. 7 is a diagram exemplarily illustrating adjustment of the stress of the subject based on previous detection values of the concentrations of phenols and indoles.
[Fig. 8] Fig. 8 is a flowchart illustrating an example operation of the stress measurement system in detection of the type and concentration of a gas.
[Fig. 9] Fig. 9 is a flowchart illustrating an example operation of the stress measurement system in determination of a prediction equation.
[Fig. 10] Fig. 10 is a flowchart illustrating the example operation of the stress measurement system in determination of a prediction equation, which is continued from Fig. 9.
[Fig. 11] Fig. 11 is a flowchart illustrating an example operation of the stress measurement system in measurement of stress.

[Fig. 12] Fig. 12 is a flowchart illustrating an example operation of the stress measurement system in correction of individual differences.

[Fig. 13] Fig. 13 is a functional block diagram illustrating an example of a stress measurement system according to another embodiment of the present disclosure. Description of Embodiments

Embodiments according to the present disclosure will be described hereinafter with reference to the drawings. The drawings are schematic illustrations.

[Example Configuration of Stress Measurement System]

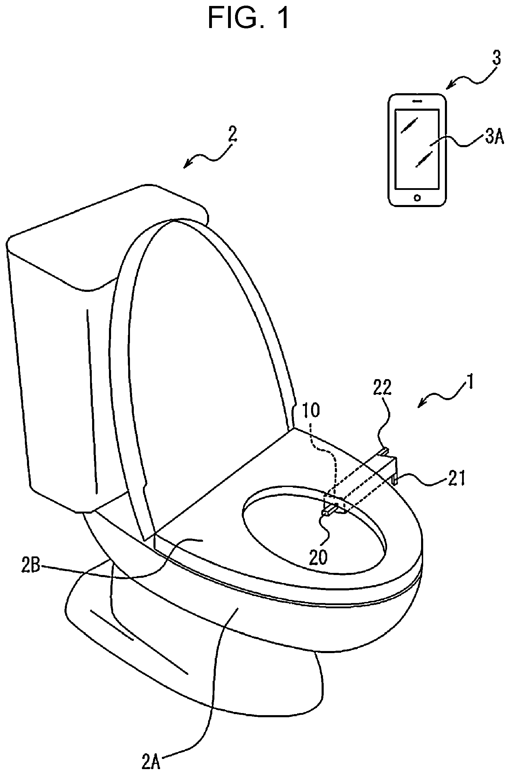

A stress measurement system 1 illustrated in Fig. 1 detects gases based on substances contained in a specimen of a subject and determines the stress level of the subject on the basis of the detection values of the gases. The specimen of the subject is a test object used to determine the stress level. The specimen of the subject may be, for example, a part of the tissue of the subject, urine, or the like. In this embodiment, the specimen of the subject is feces of the subject. The stress measurement system 1 is also a gas detection system that detects gases from the specimen to determine the stress level.

As illustrated in Fig. 1, the stress measurement system 1 is installed in, for example, a flush toilet 2. The toilet 2 includes a toilet bowl 2A and a toilet seat 2B. The stress measurement system 1 may be installed in any portion of the toilet 2. In one example, as illustrated in Fig. 1, the stress measurement system 1 may be arranged from between the toilet bowl 2A and the toilet seat 2B to the outside of the toilet 2. A portion of the stress measurement system 1 may be embedded inside the toilet seat 2B. A subject can discharge feces into the toilet bowl 2A of the toilet 2. The stress measurement system 1 can acquire a gas generated from the feces discharged into the toilet bowl 2A as a sample gas. The stress measurement system 1 can detect the concentration and the like of a plurality of gases based on specific substances contained in the sample gas. The stress measurement system 1 can transmit the detection results, the determined stress level of the subject, and so on to an electronic device 3. The specific substances are contained in the feces and are substances obtained by decomposition and excretion of raw materials for brain neurotransmitters in the body without being absorbed by the intestine. The details of the specific substances will be described below. A housing 10, a first suction hole 20, a second suction hole 21, and a discharge path 22 will be described.

The toilet 2 can be installed in a toilet room in a house, a hospital, or the like. The electronic device 3 is, for example, a smartphone used by the subject. However, the electronic device 3 is not limited to the smartphone and may be any electronic device. The electronic device 3 may be inside or outside the toilet room.

The electronic device 3 can receive the detection results from the stress measurement system 1 via wireless communication or wired communication. The electronic device 3 can display the received detection results on a display unit 3A. The display unit 3A may include a display capable of displaying characters, and a touch screen capable of detecting contact of a finger of the user (subject) or the like. The display may include a display device such as a liquid crystal display (LCD), an organic EL display (OELD: Organic Electro-Luminescence Display), or an inorganic EL display (IELD: Inorganic Electro-Luminescence Display). The detection method of the touch screen may be any method such as a capacitance method, a resistance film method, a surface acoustic wave method (or an ultrasonic method), an infrared method, an electromagnetic induction method, or a load detection method.

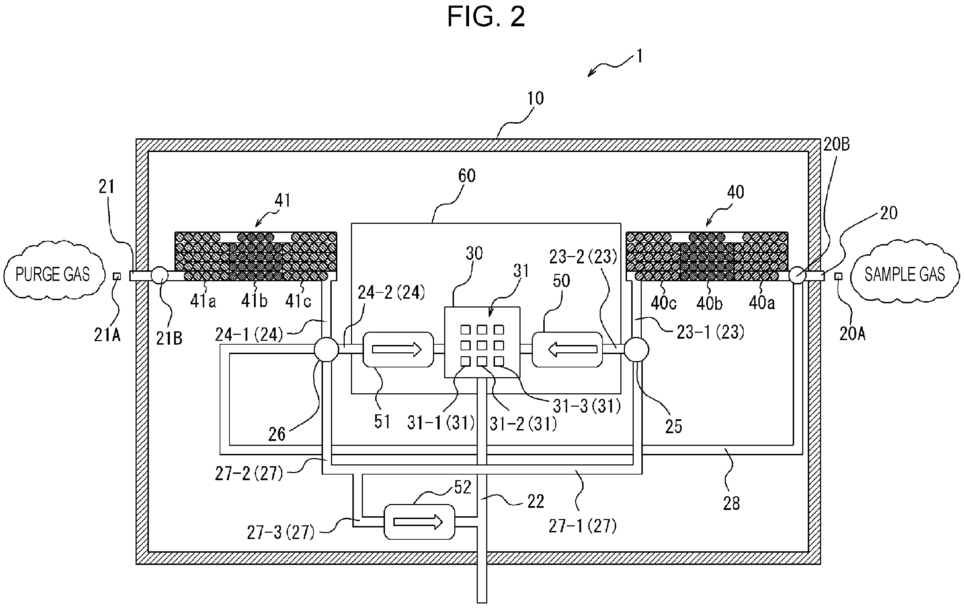

As illustrated in Fig. 2, the stress measurement system 1 includes the housing 10, the first suction hole 20, the second suction hole 21, the discharge path 22, flow paths 23 and 24, a chamber 30, a first reservoir 40, a second reservoir 41, a first supply unit 50, a second supply unit 51, and a circuit board 60. The flow path 23 includes a flow path 23-1 and a flow path 23-2. The flow path 24 includes a flow path 24-1 and a flow path 24-2. The stress measurement system 1 may include a valve 20B and a valve 21B. The stress measurement system 1 may include valves 25 and 26, a flow path 27, a flow path 28, and a third supply unit 52. The flow path 27 includes a flow path 27-1, a flow path 27-2, and a flow path 27-3.

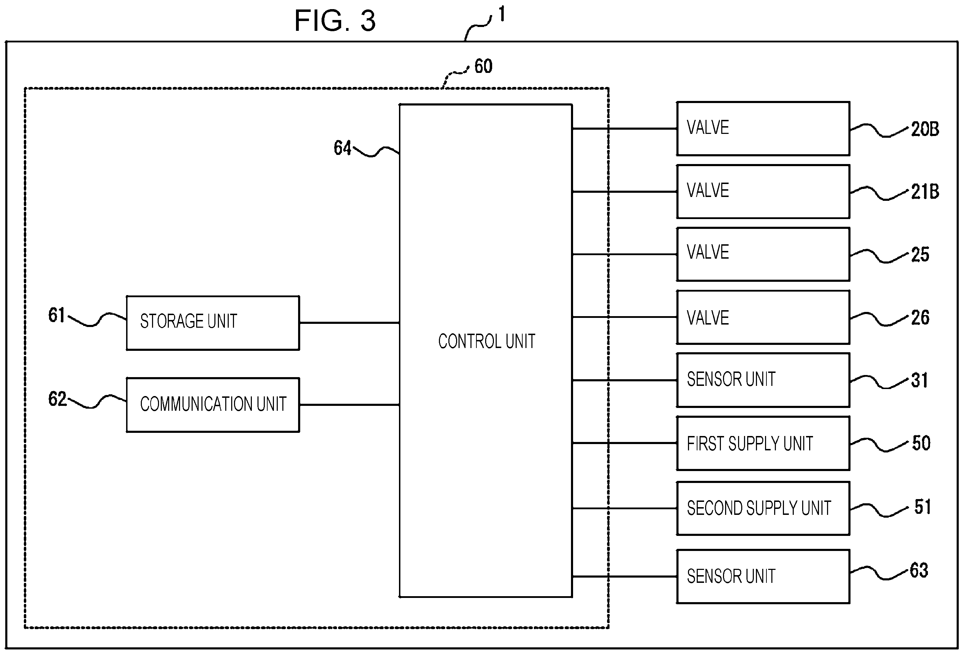

As illustrated in Fig. 3, the circuit board 60 of the stress measurement system 1 includes a storage unit 61, a communication unit 62, and a control unit 64. The stress measurement system 1 may include a sensor unit 63. The stress measurement system 1 may further include a battery, a speaker, and the like.

The housing 10 accommodates various components of the stress measurement system 1. The housing 10 may be made of any material. For example, the housing 10 may be made of a material such as metal or resin.

As illustrated in Fig. 1, the first suction hole 20 can be exposed to the inside of the toilet bowl 2A. A portion of the first suction hole 20 may be embedded in the toilet seat 2B. The first suction hole 20 sucks in a gas generated from feces discharged into the toilet bowl 2A as a sample gas. The sample gas sucked in through the first suction hole 20 is supplied to and stored in the first reservoir 40 via the valve 20B illustrated in Fig. 2. As illustrated in Fig. 1, a first end of the first suction hole 20 may be directed to the inside of the toilet bowl 2A. As illustrated in Fig. 2, a second end of the first suction hole 20 may be connected to the first reservoir 40. The first suction hole 20 may be constituted by a tubular member such as a resin tube or a metal or glass pipe.

As illustrated in Fig. 2, an air blower 20A may be disposed outside the first suction hole 20. The air blower 20A may include a fan and a motor. The air blower 20A is controlled by the control unit 64. When the motor is driven to cause the fan to rotate, the sample gas is drawn into around the first suction hole 20.

The valve 20B is located among the first suction hole 20, the first reservoir 40, and the flow path 28. The valve 20B includes a connection port connected to the first suction hole 20, a connection port connected to an inlet portion of the first reservoir 40, and a connection port connected to the flow path 28. The valve 20B may be constituted by a valve such as an electromagnetically driven valve, a piezoelectrically driven valve, or a motor-driven valve.

The control unit 64 controls the valve 20B to switch the connection state among the first suction hole 20, the first reservoir 40, and the flow path 28. For example, the control unit 64 switches the connection state among them to a state in which the first suction hole 20 and the first reservoir 40 are connected to each other, a state in which the first reservoir 40 and the flow path 28 are connected to each other, or a state in which the first suction hole 20, the first reservoir 40, and the flow path 28 are not connected to each other.

When the first suction hole 20 is to suck in the sample gas, the control unit 64 controls the valve 20B such that the first suction hole 20 and the first reservoir 40 are connected to each other. When the sample gas is stored in the first reservoir 40, the control unit 64 controls the valve 20B such that the first suction hole 20, the first reservoir 40, and the flow path 28 are not connected to each other. The first reservoir 40 is not connected to the first suction hole 20, which can reduce the probability that the sample gas in the first reservoir 40 comes into contact with the outside air.

As illustrated in Fig. 1, the second suction hole 21 can be exposed to the outside of the toilet bowl 2A. A portion of the second suction hole 21 may be embedded in the toilet seat 2B. The second suction hole 21 sucks in, for example, air (surrounding gas) in the toilet room outside the toilet bowl 2A as a purge gas. The purge gas sucked in through the second suction hole 21 is supplied to and stored in the second reservoir 41 via the valve 21B illustrated in Fig. 2. As illustrated in Fig. 1, a first end of the second suction hole 21 may be directed to the outside of the toilet 2. As illustrated in Fig. 2, a second end of the second suction hole 21 may be connected to the second reservoir 41. The second suction hole 21 may be constituted by a tubular member such as a resin tube or a metal or glass pipe.

As illustrated in Fig. 2, an air blower 21A may be disposed outside the second suction hole 21. The air blower 21A may include a fan and a motor. The air blower 21A is controlled by the control unit 64. When the motor is driven to cause the fan to rotate, the purge gas is drawn into around the second suction hole 21.

The valve 21B is located between the second suction hole 21 and the second reservoir 41. The valve 21B includes a connection port connected to the second suction hole 21, and a connection port connected to an inlet portion of the second reservoir 41. The valve 21B may be constituted by a valve such as an electromagnetically driven valve, a piezoelectrically driven valve, or a motor-driven valve.

The control unit 64 controls the valve 21B to switch the connection state between the second suction hole 21 and the second reservoir 41. For example, the control unit 64 switches the connection state between them to a state in which the second suction hole 21 and the second reservoir 41 are connected to each other or a state in which the second suction hole 21 and the second reservoir 41 are not connected to each other.

When the second suction hole 21 is to suck in the purge gas, the control unit 64 controls the valve 21B such that the second suction hole 21 and the second reservoir 41 are connected to each other. When the purge gas is stored in the second reservoir 42, the control unit 64 controls the valve 21B such that the second suction hole 21 and the second reservoir 41 are not connected to each other. The second reservoir 41 is not connected to the second suction hole 21, which can reduce the probability that the purge gas in the second reservoir 41 comes into contact with the outside air.

As illustrated in Fig. 1, a portion of the discharge path 22 can be exposed to the outside of the toilet bowl 2A. The discharge path 22 discharges the exhaust from the chamber 30 to the outside. The exhaust can contain the sample gas and the purge gas, which have been subjected to a detection process. Further, the discharge path 22 can discharge the residual gas or the like in the first reservoir 40 to the outside via the flow path 23-1, the valve 25, the flow paths 27-1 and 27-3, and the third supply unit 52. Further, the discharge path 22 can discharge the residual gas or the like in the second reservoir 41 to the outside via the flow path 24-1, the valve 26, the flow paths 27-2 and 27-3, and the third supply unit 52. The discharge path 22 may be constituted by a tubular member such as a resin tube or a metal or glass pipe.

A first end of the flow path 23-1 is connected to an outlet portion of the first reservoir 40. A second end of the flow path 23-1 is connected to the valve 25. A first end of the flow path 23-2 is connected to the valve 25. A second end of the flow path 23-2 is connected to the chamber 30 via the first supply unit 50. The flow path 23 may be constituted by a tubular member such as a resin tube or a metal or glass pipe.

A first end of the flow path 24-1 is connected to an outlet portion of the second reservoir 41. A second end of the flow path 24-1 is connected to the valve 26. A first end of the flow path 24-2 is connected to the valve 26. A second end of the flow path 24-2 is connected to the chamber 30 via the second supply unit 51. The flow path 24 may be constituted by a tubular member such as a resin tube or a metal or glass pipe.

The valve 25 is located among the flow path 23-1, the flow path 23-2, and the flow path 27-1. The valve 25 includes a connection port connected to the flow path 23-1, a connection port connected to the flow path 23-2, and a connection port connected to the flow path 27-1. The valve 25 may be constituted by a valve such as an electromagnetically driven valve, a piezoelectrically driven valve, or a motor-driven valve.

The control unit 64 controls the valve 25 to switch the connection state among the flow path 23-1, the flow path 23-2, and the flow path 27-1. For example, the control unit 64 switches the connection state among them to a state in which the flow path 23-1 and the flow path 23-2 are connected to each other or a state in which the flow path 23-1 and the flow path 27-1 are connected to each other.

The valve 26 is located among the flow path 24-1, the flow path 24-2, the flow path 27-2, and the flow path 28. The valve 26 includes a connection port connected to the flow path 24-1, a connection port connected to the flow path 24-2, a connection port connected to the flow path 27-2, and a connection port connected to the flow path 28. The valve 26 may be constituted by a valve such as an electromagnetically driven valve, a piezoelectrically driven valve, or a motor-driven valve.

The control unit 64 controls the valve 26 to switch the connection state among the flow path 24-1, the flow path 24-2, the flow path 27-2, and the flow path 28. For example, the control unit 64 switches the connection state among them to a state in which the flow path 24-1 and the flow path 24-2 are connected to each other, a state in which the flow path 24-1 and the flow path 27-2 are connected to each other, or a state in which the flow path 24-1 and the flow path 28 are connected to each other.

A first end of the flow path 27-1 is connected to the valve 25. A second end of the flow path 27-1 is connected to a first end of the flow path 27-3. A first end of the flow path 27-2 is connected to the valve 26. A second end of the flow path 27-2 is connected to the first end of the flow path 27-3. The first end of the flow path 27-3 is connected to the second end of the flow path 27-1 and the second end of the flow path 27-2. A second end of the flow path 27-3 is connected to the discharge path 22 via the third supply unit 52. The flow path 27 may be constituted by a tubular member such as a resin tube or a metal or glass pipe.

A first end of the flow path 28 is connected to the valve 20B. A second end of the flow path 28 is connected to the valve 26. The flow path 28 may be constituted by a tubular member such as a resin tube or a metal or glass pipe. The control unit 64 controls the valve 20B and the valve 26 to supply the purge gas in the second reservoir 41 to the first reservoir 40 when the flow path 24-1, the flow path 28, and the first reservoir 40 are connected to each other. At this time, the sample gas in the first reservoir 40 is pushed out to the flow path 23-1.

The chamber 30 has therein a sensor unit 31 different from the sensor unit 63 described above. The chamber 30 may have a plurality of sensor units 31. The plurality of sensor units 31 include sensor units 31-1, 31-2, and 31-3. The chamber 30 may be divided into a plurality of chambers. Each of the sensor units 31 may be disposed in a corresponding one of the resulting plurality of chambers 30. The plurality of chambers 30 may be connected to each other. Further, the chamber 30 is connected to the flow path 23-2 via the first supply unit 50. The chamber 30 is supplied with the sample gas from the flow path 23-2. The chamber 30 is also connected to the flow path 24-2 via the second supply unit 51. The chamber 30 is supplied with the purge gas from the flow path 24-2. The chamber 30 is also connected to the discharge path 22. The exhaust from the chamber 30 containing the sample gas and the purge gas after the detection process is discharged through the discharge path 22.

The sensor unit 31 is arranged in the chamber 30. The sensor unit 31 outputs a detection value corresponding to a detection result of a detection target gas to be detected. In this embodiment, the sensor unit 31 outputs a voltage corresponding to the concentration of the detection target gas to the control unit 64. In this embodiment, the sensor unit 31 outputs a plurality of detection values for a corresponding one of a plurality of detection target gases. The gases to be supplied to the chamber 30 include a detection target gas and a non-detection target gas. For example, the sample gas contains methane, hydrogen, carbon dioxide, methyl mercaptan, hydrogen sulfide, acetic acid, trimethylamine, ammonia, water, and so on. The sample gas further contains gases based on phenols including phenol, cresol, and the like, and gases based on indoles including indole, skatole, and the like. Phenol and cresol are decomposed substances of tyrosine, which is a raw material for noradrenaline, and are substances found to be contained in a fecal odor. Indole and skatole are decomposed substances of tryptophan, which is a raw material for serotonin, and are substances found to be contained in a fecal odor. The gases based on phenols and the gases based on indoles are hereinafter referred to simply as phenols and indoles, respectively. In this embodiment, the detection target gas contains at least phenols and indoles. When a gas generated from feces is a target, at least ammonia and water are non-detection target gases since ammonia is a component contained in urine and water is a component contained in urine and rinsing water.

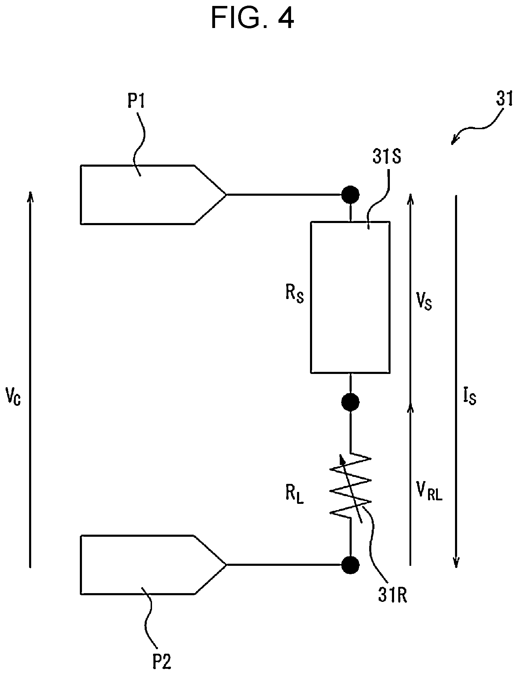

As illustrated in Fig. 4, the sensor unit 31 includes a sensor element 31S and a resistance element 31R. The sensor element 31S and the resistance element 31R are connected in series between a power supply terminal P1 and a ground terminal P2. A constant voltage value Vc is applied between the power supply terminal P1 and the ground terminal P2. Current Is having the same value flows through each of the sensor element 31S and the resistance element 31R. The value of the current $I_S$ can be determined in accordance with a resistance value $R_S$ of the sensor element 31S and a resistance value $R_L$ of the resistance element 31R. The voltage output from the sensor unit 31 may a voltage value $V_S$ across the sensor element 31S or a voltage value $V_{RL}$ across the resistance element 31R.

The power supply terminal P1 illustrated in Fig. 4 is connected to a power supply such as a battery included in the stress measurement system 1. The ground terminal P2 is connected to ground of the stress measurement system 1.

A first end of the sensor element 31S illustrated in Fig. 4 is connected to the power supply terminal P1. A second end of the sensor element 31S is connected to a first end of the resistance element 31R. The sensor element 31S is a semiconductor sensor. However, the sensor element 31S is not limited to the semiconductor sensor. For example, the sensor element 31S may be a catalytic combustion sensor, a solid electrolyte sensor, or the like.

The sensor element 31S includes a gas-sensitive portion. The gas-sensitive portion includes a metal oxide semiconductor material corresponding to the type of the sensor unit 31. Examples of the metal oxide semiconductor material include a material containing one or more selected from tin oxides (such as $SnO_2$), indium oxides (such as $In_2O_3$), zinc oxides (such as $ZnO$), tungsten oxides (such as $WO_3$), iron oxides (such as $Fe_2O_3$), and the like. Adding impurities to the metal oxide semiconductor material of the gas-sensitive portion as appropriate makes it possible to appropriately select a gas to be detected by the sensor element 31S. The sensor element 31S may further include a heater for heating the gas-sensitive portion.

When the sensor element 31S is exposed to the purge gas, oxygen contained in the purge gas can be adsorbed on a surface of the gas-sensitive portion of the sensor element 31S. The oxygen adsorbed on the surface of the gas-sensitive portion can capture free electrons on the surface of the gas-sensitive portion. When free electrons are captured by the oxygen adsorbed on the surface of the gas-sensitive portion, the resistance value $R_S$ of the sensor element 31S increases, and the voltage value $V_S$ across the sensor element 31S can increase. That is, when the purge gas is supplied to the sensor unit 31, the voltage value $V_S$ across the sensor element 31S can increase. The sum of the voltage value $V_S$ and the voltage value $V_{RL}$ has a constant value. Accordingly, when the purge gas is supplied to the sensor unit 31, the voltage value $V_{RL}$ can decrease.

When the sensor element 31S is exposed to the sample gas, the detection target gas contained in the sample gas is replaced with the oxygen adsorbed on the surface of the gas-sensitive portion of the sensor element 31S, and a reduction reaction can occur. Since the reduction reaction occurs, the oxygen adsorbed on the surface of the gas-sensitive portion can be removed. When the oxygen adsorbed on the surface of the gas-sensitive portion is removed, the resistance value $R_S$ of the sensor element 31S decreases, and the voltage value $V_S$ across the sensor element 31S can decrease. That is, when the sample gas is supplied to the sensor unit 31, the voltage value $V_S$ across the sensor element 31S can decrease in accordance with the concentration of the detection target gas contained in the sample gas. The sum of the voltage value $V_S$ and the voltage value $V_{RL}$ has a constant value. Accordingly, when the sample gas is supplied to the sensor unit 31, the voltage value $V_{RL}$ can increase in accordance with the concentration of the detection target gas contained in the sample gas.

The resistance element 31R is a variable resistance element. The resistance value $R_L$ of the resistance element 31R can be changed in accordance with a control signal from the control unit 64. The first end of the resistance element 31R is connected to the second end of the sensor element 31S. A second end of the resistance element 31R is connected to the ground terminal P2.

Adjusting the resistance value $R_L$ of the resistance element 31R can adjust the voltage value $V_S$ across the sensor element 31S. For example, when the resistance value $R_L$ is set to be equal to the resistance value $R_S$ of the sensor element 31S, the fluctuation range of the voltage value $V_S$ across the sensor element 31S can be close to a maximum value.

The first reservoir 40 is capable of storing the sample gas. An adsorbent 40a, an adsorbent 40b, and an adsorbent 40c may be placed in the first reservoir 40. Further, the sample gas may be concentrated in the first reservoir 40. In the present disclosure, "concentrating the sample gas" refers to increasing the concentration of the detection target gas contained in the sample gas. Each of the adsorbent 40a, the adsorbent 40b, and the adsorbent 40c may contain any material corresponding to the use. Each of the adsorbent 40a, the adsorbent 40b, and the adsorbent 40c may contain, for example, at least one of activated charcoal, silica gel, zeolite, an MOF (Metal Organic Frameworks) material, a molecularly imprinted material, or molecular sieve. The adsorbent 40a, the adsorbent 40b, and the adsorbent 40c may be of a plurality of types or may contain a porous material.

The adsorbent 40a may contain, for example, at least one of silica gel, zeolite, or the like. The adsorbent 40b may contain, for example, at least one of activated charcoal, an MOF material, a molecularly imprinted material, molecular sieve, or the like. The adsorbent 40c may contain, for example, at least one of activated charcoal, an MOF material, a molecularly imprinted material, molecular sieve, or the like. The configurations of the adsorbent 40a, the adsorbent 40b, and the adsorbent 40c are not limited to those described above, and may be appropriately changed according to the polarity of gas molecules to be adsorbed. In this embodiment, one of the adsorbent 40b and the adsorbent 40c adsorbs phenols. The other of the adsorbent 40b and the adsorbent 40c adsorbs indoles. Phenols and indoles typically have boiling points higher than room temperature (15 to 25°C) and may be contained in the sample gas only in the order of ppb. In this embodiment, in the first reservoir 40, the adsorbent 40b and the adsorbent 40c selectively adsorb phenols and indoles, and desorb phenols and indoles when a predetermined temperature is reached by heating with the heater. That is, in the first reservoir 40, phenols and indoles serving as detection target gases that are selectively adsorbed by the adsorbent 40b and the adsorbent 40c are heated and desorbed to increase the concentrations of the detection target gases. Accordingly, the sample gas is concentrated in the first reservoir 40. The adsorbent 40a further adsorbs noise gas, which is not phenols or indoles. That is, noise gas is removed in the first reservoir 40. The concentration and noise gas removal, which are performed in the first reservoir 40, enable the sensor unit 31 to improve the detection accuracy of phenols and indoles. In the first reservoir 40, only one of the detection target gas concentration and the noise gas removal may be executed.

Specifically, the sample gas concentration described above is performed by, for example, sequentially performing the following steps (1) to (3).

(1) The sample gas is adsorbed by the adsorbent 40a or the adsorbent 40b at room temperature or a higher temperature while the first suction hole 20, the first reservoir 40, the flow path 23-1, the flow path 27-1, the flow path, 27-3, the third supply unit 52, and the discharge path 22 are connected to each other (communicate).
(2) The adsorbent 40a or the adsorbent 40b is heated at a temperature higher than the temperature in step (1) while both the valve 20B and the valve 25 are closed, to desorb the sample gas.
(3) While the second reservoir 41, the flow path 24-1, the flow path 28, the first reservoir 40, the flow path 23-1, the flow path 23-2, the first supply unit 50, the chamber 30, and the discharge path 22 are connected to each other (communicate), a concentrated gas generated in the first reservoir 40 in step (2) described above is fed into the chamber 30.

In the first reservoir 40, the adsorbent 40a may be divided by a wall or the like. Dividing the adsorbent 40a can lengthen the flow path of the gas in the first reservoir 40. The flow path of the gas in the first reservoir 40 is lengthened, which can lengthen the time during which the gas and the adsorbent 40a are in contact with each other. Likewise, in the first reservoir 40, the adsorbent 40b may be divided by a wall or the like. Dividing the adsorbent 40b can lengthen the time during which the gas and the adsorbent 40b are in contact with each other in the first reservoir 40. Likewise, in the first reservoir 40, the adsorbent 40c may be divided by a wall or the like. Dividing the adsorbent 40c can lengthen the time during which the gas and the adsorbent 40c are in contact with each other in the first reservoir 40.

The adsorbent 40a may be placed on the side of the first reservoir 40 where the first reservoir 40 is connected to the first suction hole 20. The adsorbent 40c may be placed on the side of the first reservoir 40 where the first reservoir 40 is connected to the flow path 23-1. The adsorbent 40b may be placed between the adsorbent 40a and the adsorbent 40c in the first reservoir 40.

The first reservoir 40 may be formed by a tank or the like having a rectangular parallelepiped shape, a cylindrical shape, a bag shape, or a shape that fits in a gap between various components housed inside the housing 10. The first reservoir 40 may be provided with a heater for heating at least one of an inner wall of the first reservoir 40, the adsorbent 40a, the adsorbent 40b, or the adsorbent 40c.

The entire first reservoir 40 may be divided by a wall or the like. Dividing the entire first reservoir 40 allows the flow path of the gas to have a small cross-sectional area relative to the volume of the flow path of the gas in the first reservoir 40. The cross-sectional area of the flow path of the gas is small relative to the volume of the flow path of the gas. As a result, when the sample gas is to be pushed out into the chamber 30 from the first reservoir 40, the contact area between the gas flowing into the first reservoir 40 from the valve 20B and the sample gas stored in the first reservoir 40 can be small. The contact area between the gas flowing into the first reservoir 40 from the valve 20B and the sample gas stored in the first reservoir 40 is small. As a result, the gas flowing into the first reservoir 40 from the valve 20B is less likely to be mixed with the sample gas in the first reservoir 40.

The second reservoir 41 is capable of storing the purge gas. An adsorbent 41a, an adsorbent 41b, and an adsorbent 41c may be placed in the second reservoir 41. Each of the adsorbent 41a, the adsorbent 41b, and the adsorbent 41c may contain any material corresponding to the use. Each of the adsorbent 41a, the adsorbent 41b, and the adsorbent 41c may contain, for example, at least one of activated charcoal, silica gel, zeolite, an MOF material, a molecularly imprinted material, or molecular sieve. The adsorbent 41a, the adsorbent 41b, and the adsorbent 41c may be of a plurality of types or may contain a porous material.

The adsorbent 41a may contain, for example, at least one of silica gel, zeolite, or the like. The adsorbent 41b may contain, for example, at least one of activated charcoal, an MOF material, a molecularly imprinted material, molecular sieve, or the like. The adsorbent 41c may contain, for example, at least one of activated charcoal, an MOF material, a molecularly imprinted material, molecular sieve, or the like. The configurations of the adsorbent 41a, the adsorbent 41b, and the adsorbent 41c are not limited to those described above, and may be appropriately changed according to the polarity of gas molecules to be adsorbed. For example, one of the adsorbent 41b and the adsorbent 41c may adsorb phenols. The other of the adsorbent 41b and the adsorbent 41c may adsorb indoles. The adsorbent 41a may adsorb noise gas, which is not phenols or indoles.

In the second reservoir 41, the adsorbent 41a may be divided by a wall or the like. Dividing the adsorbent 41a can lengthen the flow path of the gas in the second reservoir 41. The flow path of the gas in the second reservoir 41 is lengthened, which can lengthen the time during which the gas and the adsorbent 41a are in contact with each other. Likewise, in the second reservoir 41, the adsorbent 41b may be divided by a wall or the like. Dividing the adsorbent 41b can lengthen the time during which the gas and the adsorbent 41b are in contact with each other in the second reservoir 41. Likewise, in the second reservoir 41, the adsorbent 41c may be divided by a wall or the like. Dividing the adsorbent 41c can lengthen the time during which the gas and the adsorbent 41c are in contact with each other in the second reservoir 41.

The adsorbent 41a may be placed on the side of the second reservoir 41 where the second reservoir 41 is connected to the second suction hole 21. The adsorbent 41c may be placed on the side of the second reservoir 41 where the second reservoir 41 is connected to the flow path 24-1. The adsorbent 41b may be placed between the adsorbent 41a and the adsorbent 41c in the second reservoir 41.

The second reservoir 41 may be formed by a tank or the like having a rectangular parallelepiped shape, a cylindrical shape, a bag shape, or a shape that fits in a gap between various components housed inside the housing 10. The second reservoir 41 may be provided with a heater for heating at least one of an inner wall of the second reservoir 41, the adsorbent 41a, the adsorbent 41b, or the adsorbent 41c.

The entire second reservoir 41 may be divided by a wall or the like. Dividing the entire second reservoir 41 allows the flow path of the gas to have a small cross-sectional area relative to the volume of the flow path of the gas in the second reservoir 41. The cross-sectional area of the flow path of the gas is small relative to the volume of the flow path of the gas. As a result, when the purge gas is to be pushed out into the chamber 30 from the second reservoir 41, the contact area between the gas flowing into the second reservoir 41 from the valve 21B and the purge gas stored in the second reservoir 41 can be small. The contact area between the gas flowing into the second reservoir 41 from the valve 21B and the purge gas stored in the second reservoir 41 is small. As a result, the gas flowing into the second reservoir 41 from the valve 21B is less likely to be mixed with the purge gas in the second reservoir 41. With this configuration, for example, if a gas near the second suction hole 21 is contaminated, the contaminated gas is less likely to be mixed with the purge gas in the second reservoir 41.

The first supply unit 50 is attached to the flow path 23-2. The first supply unit 50 is capable of supplying the sample gas stored in the first reservoir 40 to the chamber 30 when the flow path 23-1 and the flow path 23-2 are connected to each other. The first supply unit 50 is driven or stopped under the control of the control unit 64. An arrow in the first supply unit 50 in Fig. 2 indicates the direction in which the first supply unit 50 sends the sample gas. The first supply unit 50 may be constituted by a piezoelectric pump, a motor pump, or the like.

The second supply unit 51 is attached to the flow path 24-2. The second supply unit 51 is capable of supplying the purge gas stored in the second reservoir 41 to the chamber 30 when the flow path 24-1 and the flow path 24-2 are connected to each other. The second supply unit 51 is driven or stopped under the control of the control unit 64. An arrow in the second supply unit 51 in Fig. 2 indicates the direction in which the second supply unit 51 sends the purge gas. The second supply unit 51 may be constituted by a piezoelectric pump, a motor pump, or the like.

The third supply unit 52 is attached to the flow path 27-3. The third supply unit 52 is capable of supplying the residual gas or the like in the first reservoir 40 to the discharge path 22 when the flow path 23-1 and the flow path 27-1 are connected to each other. Further, the third supply unit 52 is capable of supplying the residual gas or the like in the second reservoir 41 to the discharge path 22 when the flow path 24-1 and the flow path 27-2 are connected to each other. The third supply unit 52 is driven or stopped under the control of the control unit 64. An arrow in the third supply unit 52 in Fig. 2 indicates the direction in which the third supply unit 52 sends the residual gas or the like. The third supply unit 52 may be constituted by a piezoelectric pump, a motor pump, or the like.

The third supply unit 52 also has a function of drawing the sample gas and the purge gas into the first reservoir 40 and the second reservoir 41, respectively. The third supply unit 52 is capable of supplying the sample gas from the first suction hole 20 to the first reservoir 40 when the first suction hole 20 and the first reservoir 40 are connected to each other and the flow path 23-1 and the flow path 27-1 are connected to each other. Further, the third supply unit 52 is capable of supplying the purge gas from the second suction hole 21 to the second reservoir 41 when the second suction hole 21 and the second reservoir 41 are connected to each other and the flow path 24-1 and the flow path 27-2 are connected to each other.

As described above, the circuit board 60 includes the storage unit 61, the communication unit 62, the control unit 64, and so on (see Fig. 3). The storage unit 61 is constituted by, for example, a semiconductor memory, a magnetic memory, or the like. The storage unit 61 stores various kinds of information and a program for activating the stress measurement system 1. The storage unit 61 may function as a work memory.

The storage unit 61 stores, for example, a multiple regression analysis algorithm. The storage unit 61 stores, for example, a model equation in the multiple regression analysis (for example, model equation (2) described below). The storage unit 61 stores information related to a standard gas described below. The storage unit 61 stores, for example, information related to a prediction equation described below (such as information on prediction equation (1) described below), which is determined or updated in the stress measurement system 1 or an external server.

The communication unit 62 communicates with the electronic device 3 configured to present the stress level of the subject, which is measured by the control unit 64, to the subject via, for example, display on the display unit 3A or via sound. The communication unit 62 may be capable of communicably communicating with an external server. The communication method used when the communication unit 62 communicates with the electronic device 3 and the external server may be a short-range wireless communication standard, a wireless communication standard for connecting to a mobile phone network, or a wired communication standard. The short-range wireless communication standard may include, for example, WiFi (registered trademark), Bluetooth (registered trademark), infrared, NFC (Near Field Communication), and the like. The wireless communication standard for connecting to a mobile phone network may include, for example, LTE (Long Term Evolution) or a fourth generation or higher mobile communication system, and the like. Alternatively, the communication method used when the communication unit 62 communicates with the electronic device 3 and the external server may be, for example, a communication standard such as LPWA (Low Power Wide Area) or LPWAN (Low Power Wide Area Network).

The sensor unit 63 may include at least any one of an image camera, a personal identification switch, an infrared sensor, a pressure sensor, or the like. The sensor unit 63 outputs a detection result to the control unit 64.

For example, when the sensor unit 63 includes an infrared sensor, the sensor unit 63 detects reflected light from an object irradiated with infrared radiation from the infrared sensor, thereby being able to detect that the subject has entered the toilet room. The sensor unit 63 outputs, as a detection result, a signal indicating that the subject has entered the toilet room to the control unit 64.

For example, when the sensor unit 63 includes a pressure sensor, the sensor unit 63 detects a pressure applied to the toilet seat 2B illustrated in Fig. 1, thereby being able to detect that the subject has sit on the toilet seat 2B. The sensor unit 63 outputs, as a detection result, a signal indicating that the subject has sit on the toilet seat 2B to the control unit 64.

For example, when the sensor unit 63 includes a pressure sensor, the sensor unit 63 detects a reduction in the pressure applied to the toilet seat 2B illustrated in Fig. 1, thereby being able to detect that the subject has risen from the toilet seat 2B. The sensor unit 63 outputs, as a detection result, a signal indicating that the subject has risen from the toilet seat 2B to the control unit 64.

For example, when the sensor unit 63 includes an image camera, a personal identification switch, and the like, the sensor unit 63 collects data, such as a face image, the sitting height, and the weight. The sensor unit 63 identifies and detects a person from the collected data. The sensor unit 63 outputs, as a detection result, a signal indicating the identified person to the control unit 64.

For example, when the sensor unit 63 includes a personal identification switch and the like, the sensor unit 63 identifies (detects) a person in response to an operation of the personal identification switch. In this case, personal information may be registered (stored) in the storage unit 61 in advance. The sensor unit 63 outputs, as a detection result, a signal indicating the identified person to the control unit 64.

The control unit 64 includes one or more processors. The one or more processors may include at least any one of a general-purpose processor that reads a specific program to execute a specific function, and a dedicated processor dedicated to a specific process. The dedicated processor may include an application specific IC (ASIC; Application Specific Integrated Circuit). The one or more processors may include a programmable logic device (PLD; Programmable Logic Device). The PLD may include an FPGA (Field-Programmable Gate Array). The control unit 64 may include at least any one of an SoC (System-on-a-chip) and an SiP (System-in-a-Package) with which the one or more processors cooperate. The control unit 64 may perform computation described below (such as a process for determining the type and concentration of a gas, or a stress measurement process) in accordance with a program.

<Process for Detecting type and Concentration of Gas>

For example, the control unit 64 connects the flow path 24-1 and the flow path 24-2 to each other and drives the second supply unit 51 to allow the purge gas to be stored in the second reservoir 41. Then, the control unit 64 causes the purge gas stored in the second reservoir 41 to be supplied to the chamber 30. If the purge gas near the second suction hole 21 is contaminated, the control unit 64 may close the valve 21B to prevent the purge gas from being introduced into the second reservoir 41. For example, when the second reservoir 41 is made of resin or has a pleated structure or the like such that the internal volume thereof is changeable, the control unit 64 may perform a process for preventing the purge gas from being introduced into the second reservoir 41, as described above.

For example, the control unit 64 connects the flow path 23-1 and the flow path 23-2 to each other and drives the first supply unit 50 to allow the sample gas to be stored in the first reservoir 40. Then, the control unit 64 causes the sample gas stored in the first reservoir 40 to be supplied to the chamber 30. To prevent mixture of the sample gas near the first suction hole 20 and the sample gas already stored in the first reservoir 40, the control unit 64 may close the valve 20B to prevent the sample gas from being introduced into the first reservoir 40. At this time, the control unit 64 may control the valve 20B and the valve 26 to connect the first reservoir 40 to the second reservoir 41 via the flow path 28 and the flow path 24-1 such that the purge gas stored in the second reservoir 41 can be supplied to the first reservoir 40. For example, when the first reservoir 40 is made of resin or has a pleated structure or the like such that the internal volume thereof is changeable, the control unit 64 may perform a process for preventing the sample gas from being introduced into the first reservoir 40, as described above.

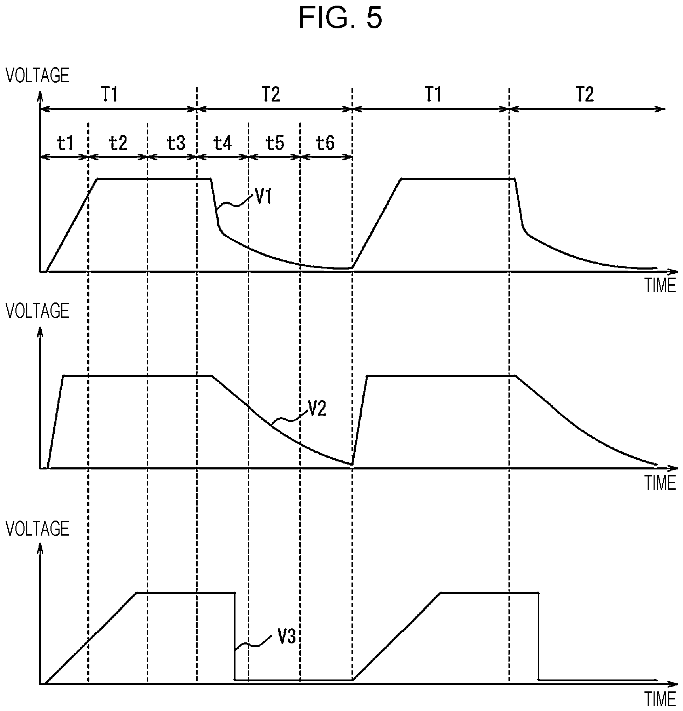

The control unit 64 controls the second supply unit 51 and the first supply unit 50 to alternately supply the purge gas and the sample gas to the chamber 30. The control unit 64 acquires a voltage waveform output by the sensor unit 31 in the chamber 30. For example, the control unit 64 acquires the voltage value $V_{RL}$ across the resistance element 31R to acquire a voltage waveform illustrated in Fig. 5.

Fig. 5 is a diagram illustrating an example of voltage waveforms of sensor units 31. In Fig. 5, the horizontal axis represents time. In Fig. 5, the vertical axis represents voltage. A voltage value indicated by a voltage waveform V1 is the voltage value $V_{RL}$ across the resistance element 31R of the sensor unit 31-1. A voltage value indicated by a voltage waveform V2 is the voltage value $V_{RL}$ across the resistance element 31R of the sensor unit 31-2. A voltage value indicated by a voltage waveform V3 is the voltage value $V_{RL}$ across the resistance element 31R of the sensor unit 31-3.

A first period T1 is a period during which the sample gas stored in the first reservoir 40 is supplied to the chamber 30. When the sample gas is supplied to the sensor unit 31, the voltage value $V_{RL}$ of the resistance element 31R can increase in accordance with the concentration of the detection target gas contained in the sample gas. In the first period T1, accordingly, the voltage values indicated by the voltage waveforms V1 to V3 increase.

A second period T2 is a period during which the purge gas stored in the second reservoir 41 is supplied to the chamber 30. When the purge gas is supplied to the sensor unit 31, the voltage value $V_{RL}$ of the resistance element 31R can decrease. In the second period T2, accordingly, the voltage values indicated by the voltage waveforms V1 to V3 decrease.

The control unit 64 detects the type and concentration of a gas contained in the sample gas on the basis of multiple regression analysis using as an explanatory variable a characteristic of a voltage waveform output by the sensor unit 31. Examples of the characteristic of the voltage waveform that can be an explanatory variable include a slope, an average value, and a median value of the voltage waveform in a predetermined section, differences between these numerical values, and the ratios of these numerical values of different sensor units 31. In the example illustrated in Fig. 5, sections t1 to t6 can be predetermined sections. The sections t1 to t6 have the same width. However, the sections t1 to t6 may have different widths. The slope of a voltage waveform in the section t1 can be one of the explanatory variables. The respective slopes of the voltage waveforms V1 to V3 in the section t1 are represented by "explanatory variable x11", "explanatory variable x12", and "explanatory variable x13", respectively. The slope of a voltage waveform in the section t2 can be one of the explanatory variables. The respective slopes of the voltage waveforms V1 to V3 in the section t2 are represented by "explanatory variable x21", "explanatory variable x22", and "explanatory variable x23", respectively. The average value of a voltage waveform in the section t3 can be one of the explanatory variables. The respective average values of the voltage waveforms V1 to V3 in the section t3 are represented by "explanatory variable x31", "explanatory variable x32", and "explanatory variable x33", respectively. The slope of a voltage waveform in the section t4 can be one of the explanatory variables. The respective slopes of the voltage waveforms V1 to V3 in the section t4 are represented by "explanatory variable x41", "explanatory variable x42", and "explanatory variable x43", respectively. The slope of a voltage waveform in the section t5 can be one of the explanatory variables. The respective slopes of the voltage waveforms V1 to V3 in the section t5 are represented by "explanatory variable x51", "explanatory variable x52", and "explanatory variable x53", respectively. The average value of a voltage waveform in the section t6 can be one of the explanatory variables. The respective average values of the voltage waveforms V1 to V3 in the section t6 are represented by "explanatory variable x61", "explanatory variable x62", and "explanatory variable x63", respectively.

The control unit 64 detects the type and concentration of a gas contained in the sample gas using a prediction equation determined by the multiple regression analysis and an explanatory variable used in the prediction equation among the explanatory variables. For example, the control unit 64 detects the type and concentration of a gas contained in the sample gas using prediction equation (1) below. Prediction equation (1) is an example of a prediction equation for predicting the concentration of a predetermined gas. Prediction equation (1) is determined by multiple regression analysis using a mixed gas whose gas composition is known. A process for determining prediction equation (1) will be described below.

[Math. 1]

$$y_1 = A \times x11 + B \times x22 + C \times x33 + D$$

prediction equation (1)

In prediction equation (1), the concentration $y_1$ is the concentration of a predetermined gas. The coefficients A, B, and C are regression coefficients of the explanatory variables x11, x22, and x33, respectively. The constant D is a constant term. Among the explanatory variables x11 to x13, x21 to x23, x31 to x33, x41 to x43, x51 to x53, and x61 to x63 described above, the explanatory variables x11, x22, and x33 are used in prediction equation (1).

The control unit 64 may acquire information related to the prediction equation from the outside via the storage unit 61 or the communication unit 62. The information related to the prediction equation may include information on the prediction equation, information on an explanatory variable used in the prediction equation, information on a predetermined interval, information on computation for acquiring the explanatory variable, and the like. The predetermined interval is an interval used to divide a voltage waveform into a plurality of sections. The predetermined interval corresponds to the width of the sections t1 to t6 illustrated in Fig. 5. For example, in the case of prediction equation (1), the information related to the prediction equation can include information on prediction equation (1), information on the explanatory variables x11, x22, and x33 used in prediction equation (1), information on the predetermined interval, and information on computation for acquiring the explanatory variables x11, x22, and x33. For example, upon acquiring the information related to the prediction equation, the control unit 64 divides the voltage waveforms illustrated in Fig. 5 by the predetermined interval along the time axis into the sections t1 to t6 as a plurality of sections. Further, the control unit 64 calculates the slope of the voltage waveform V1 in the section t1 illustrated in Fig. 5, based on the information on computation for acquiring the explanatory variable, to acquire the explanatory variable x11. Further, the control unit 64 calculates the average value of the voltage waveform V2 in the section t2 illustrated in Fig. 5 to acquire the explanatory variable x22. Further, the control unit 64 calculates the average value of the voltage waveform V3 in the section t3 illustrated in Fig. 5 to acquire the explanatory variable x33. The control unit 64 substitutes the explanatory variables x11, x22, and x33 into prediction equation (1) described above to detect the concentration y1 of the predetermined gas.

Regarding the widths of the sections corresponding to the explanatory variables, in the example illustrated in Fig. 5, the sections t1 to t6 may not have the same width. For example, the sections corresponding to the explanatory variables may have different widths. Some of the sections corresponding to the explanatory variables may overlap. The sections corresponding to the explanatory variables may include a subdivided section for acquiring a certain explanatory variable. The settings of a section corresponding to an appropriate explanatory variable may be appropriately selected in advance according to data of a voltage waveform output by the sensor unit 31, the time interval at which the voltage waveform is acquired, and the magnitude or frequency of noise included in the voltage waveform. Not all of the data of the voltage waveform output by the sensor unit 31 may be used to detect the type and concentration of a gas. For example, data of only a necessary portion of the voltage waveform output by the sensor unit 31, as appropriate, which is obtained by, for example, removing unnecessary portions, may be used to detect the type and concentration of a gas.

The control unit 64 may use a different prediction equation in accordance with a type of gas. Using a different prediction equation corresponding to a type of gas, the control unit 64 can detect the concentration for each type of gas contained in the sample gas. In other words, the control unit 64 can detect the type and concentration of a gas contained in the sample gas.

The control unit 64 may transmit the detected type and concentration of the gas to the electronic device 3 via the communication unit 62. Further, after the detection process is completed, the control unit 64 may connect the flow path 23-1 and the flow path 27-1 to each other and drive the third supply unit 52 to discharge the residual gas in the first reservoir 40 from the discharge path 22. After the detection process is completed, furthermore, the control unit 64 may connect the flow path 24-1 and the flow path 27-2 to each other and drive the third supply unit 52 to discharge the residual gas in the second reservoir 41 from the discharge path 22.

<Prediction Equation Determination Process>

The following prediction equation determination process may be performed before shipment, during maintenance, or the like of the stress measurement system 1.

The control unit 64 causes the purge gas to be sucked in through the second suction hole 21 in a way similar to that described above in accordance with a program incorporated therein in advance or when a control signal for providing an instruction to suck in the purge gas is received from the outside via the communication unit 62. The control signal for providing an instruction to suck in the purge gas can be transmitted to the stress measurement system 1 when a prediction equation is to be determined before shipment or the like of the stress measurement system 1. The control unit 64 causes the purge gas to be sucked in through the second suction hole 21 to store the purge gas in the second reservoir 41.

The control unit 64 causes the sample gas to be sucked in through the first suction hole 20 in a way similar to that described above in accordance with a program incorporated therein in advance or when a control signal for providing an instruction to suck in the sample gas is received from the outside via the communication unit 62. The control signal for providing an instruction to suck in the sample gas can be transmitted to the stress measurement system 1 when a prediction equation is to be determined before shipment or the like of the stress measurement system 1. The control unit 64 causes the sample gas to be sucked in through the first suction hole 20 to store the sample gas in the first reservoir 40. In the prediction equation determination process, a mixed gas whose gas composition is known is used as the sample gas. That is, a mixed gas whose gas composition is known is stored in the first reservoir 40. The mixed gas whose gas composition is known is hereinafter referred to also as "standard gas".

The control unit 64 acquires a model equation in the multiple regression analysis from the outside via the storage unit 61 or the communication unit 62. For example, the control unit 64 acquires model equation (2) below.
[Math. 2]

$$y_n = \sum_i \sum_j E_{ijn} \times X_{ij} + F$$

model equation (2)

In model equation (2), n, i, and j are natural numbers. n corresponds to a type of gas. In the following, the gas corresponding to n is also referred to as "gas n". i corresponds to a section corresponding to an explanatory variable. In the following, the section corresponding to i is also referred to as "section i". j corresponds to any one of a plurality of sensor units 31. In the following, the sensor unit 31 corresponding to j is also referred to as "sensor unit 31j". The concentration $y_n$ is the concentration of the gas n. The explanatory variable ij is the explanatory variable for the voltage waveform of the sensor unit 31j corresponding to the section i. The coefficient $E_{ijn}$ is the coefficient of the explanatory variable ij for the gas n. The error F is an error term.

The control unit 64 acquires information related to the standard gas from the outside via the storage unit 61 or the communication unit 62. The information related to the standard gas includes information on the type and concentration of a gas contained in the standard gas, and information related to acquisition of an explanatory variable. For example, in the case of model equation (2), the information on the type and concentration of a gas is information on the type and the concentration $y_n$ of the gas n. For example, in the case of model equation (2), the information related to acquisition of an explanatory variable can include information on the section i, and information on computation for acquiring the explanatory variable ij from the section i of the sensor unit 31j.

The control unit 64 alternately supplies the purge gas and the sample gas to the chamber 30 in a way similar to that described above to acquire a voltage waveform output by the sensor unit 31 in the chamber 30. The control unit 64 executes machine learning with training data on the voltage waveform of the sensor unit 31 to acquire an effective explanatory variable and a regression coefficient in model equation (2). The control unit 64 acquires an effective explanatory variable and a regression coefficient to determine a prediction equation for the gas n.

For example, in the case of the concentration $y_1$ (n = 1) of a predetermined gas, the control unit 64 acquires the explanatory variables $x_{11}$, $x_{22}$, and $x_{33}$ as effective explanatory variables. The control unit 64 acquires the coefficient A as the coefficient $E_{111}$ of the explanatory variable $x_{11}$. The control unit 64 acquires the coefficient B as the coefficient $E_{221}$ of the explanatory variable $x_{11}$. The control unit 64 acquires the coefficient C as the coefficient $E_{331}$ of the explanatory variable $x_{33}$. The control unit 64 acquires the constant D as the error F. The control unit 64 acquires the effective explanatory variables $x_{11}$, 22, and $x_{33}$, the coefficients A, B, and C, and the constant D to determine prediction equation (1) described above of the concentration $y_1$ of the predetermined gas. The control unit 64 may store the effective explanatory variables $x_{11}$, $x_{22}$, and $x_{33}$, the coefficients A, B, and C, and the constant D in the storage unit 61.

The control unit 64 can determine a different prediction equation in accordance with a type of gas. The control unit 64 does not need to learn all of the acquired data of the voltage waveform of the sensor unit 31. The settings of a section corresponding to an appropriate explanatory variable may be appropriately selected in advance according to data of the voltage waveform output by the sensor unit 31, the time interval at which the voltage waveform is acquired, and the magnitude or frequency of noise included in the voltage waveform. Alternatively, multiple regression analysis including all possible explanatory variables may be used to extract more effective explanatory variables.

<Stress Measurement Process>

The brain and the intestine communicate and interact with each other (via brain neurotransmitters or hormones). For example, it is known that stress can cause symptoms such as irritable bowel syndrome. It is also known that some of the substances produced from proteins and lipids in ingested foods in the intestine are transported to the brain and become raw materials for brain neurotransmitters. The raw material for noradrenaline, which is a brain neurotransmitter, is tyrosine. The raw material for serotonin, which is a brain neurotransmitter, is tryptophan. For example, when tyrosine in the intestine is transported from the inside of the intestine to the brain via a derivative and the amount of tyrosine in the intestine decreases, the amount of noradrenaline in the brain increases. When the amount of tyrosine in the intestine decreases, the amount of phenols in the intestine, which are decomposed substances thereof, also decreases. When tryptophan in the intestine is transported from the inside of the intestine to the brain via a derivative and the amount of tryptophan in the intestine decreases, the amount of serotonin in the brain increases. When the amount of tryptophan in the intestine decreases, the amount of indoles in the intestine, which are decomposed substances thereof, also decreases.

Stress upsets the balance between sympathetic nerve activity and parasympathetic nerve activity. That is, it is possible to measure the stress level from the sympathetic nerve activity and the parasympathetic nerve activity. For example, increasing the stress level of the subject activates the sympathetic nervous system, and the sympathetic nervous system dominates over the parasympathetic nervous system. At this time, tyrosine, which is a raw material for noradrenaline, decreases in the intestine, and phenols also decrease accordingly. Decreasing the stress level of the subject activates the parasympathetic nervous system. Then, tryptophan, which is a raw material for serotonin, decreases, and indoles also decrease accordingly.

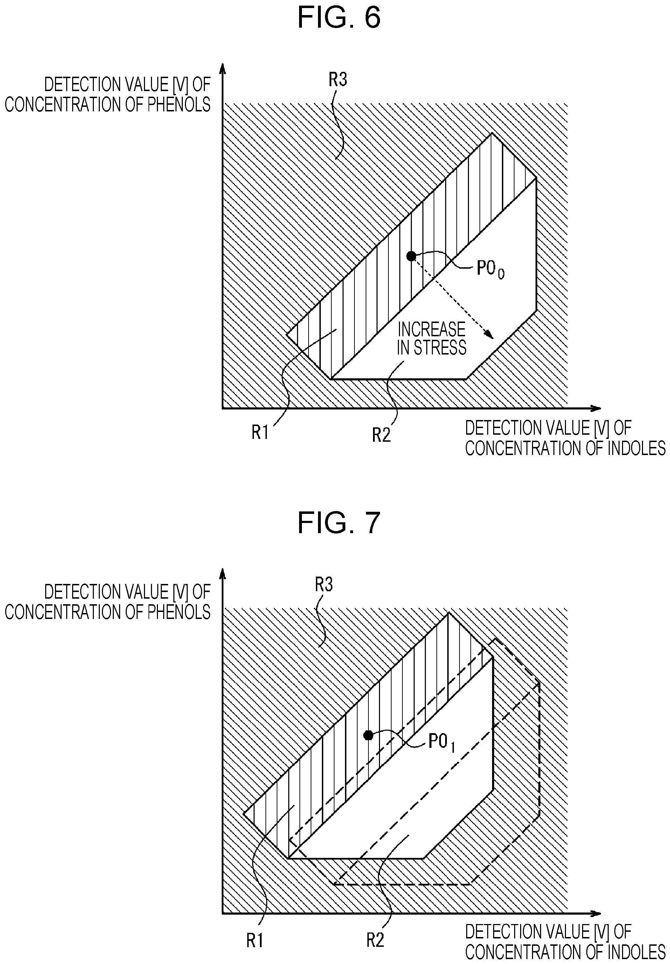

Fig. 6 is a diagram exemplarily illustrating the relationship between the detection values of the concentrations of phenols and indoles, which are detection target gases, and the stress level of the subject. In Fig. 6, the horizontal axis represents the detection value of the concentration of indoles, that is, the voltage corresponding to the concentration of indoles. In Fig. 6, the vertical axis represents the detection value of the concentration of phenols, that is, the voltage corresponding to the concentration of phenols. When the stress level of the subject is sufficiently low, the balance between the amount of phenols and the amount of indoles in the intestine is achieved, and the detection value of the concentration of indoles and the detection value of the concentration of phenols are included in a region R1. The region R1 is defined as a certain range centered on a reference value POo determined as an actual measurement value or a calculated value of a person in a stress-free state. As described below, the reference value POo may be replaced with a reference value $PO_1$ that takes individual differences for each subject into account. When the stress level of the subject increases, phenols decrease and indoles increase. As a result, the detection value of the concentration of indoles and the detection value of the concentration of phenols are included in a region R2. The region R2 is also defined as a range extending from the reference value POo determined as an actual measurement value or a calculated value of a person in a stress-free state. In Fig. 6, the region R2 is a region surrounded by a line connecting the maximum value of the concentration of indoles in the region R1 and the minimum value of the concentration of phenols in the region R1, a line extending from the maximum value of the concentration of indoles in the region R1 in a direction in which the concentration of phenols decreases, and a line extending from the minimum value of the concentration of phenols in the region R1 in a direction in which the concentration of indoles increases. The regions R1 and R2 may be determined by a method such as deep learning. In Fig. 6, as indicated by a dotted-line arrow, as the stress level of the subject increases, the concentration of indoles relatively increases. It is therefore possible to measure the stress level of the subject from the ratio of the concentrations of phenols and indoles. When the detection value of the concentration of indoles and the detection value of the concentration of phenols are included in a region (region R3) other than the region R1 and the region R2, the subject may be in an abnormal state. That is, when the combination of the concentrations of phenols and indoles is in the region R3, the subject may have, for example, too much serotonin, serotonin syndrome, mania, schizophrenia, insomnia, or depression.

The control unit 64 acquires detection values of the concentrations of phenols and indoles among the types and concentrations of gases detected according to prediction equation (1) described above. To extract only the relationship between noradrenaline and serotonin, the control unit 64 may acquire the detection values of the concentrations of tyrosine and tryptophan.

The control unit 64 determines a combination of the detection value of the concentration of phenols and the detection value of the concentration of indoles. The control unit 64 determines whether the determined combination can be included in the region R1 and the region R2. When the determined detection value of the concentration of phenols and the determined detection value of the concentration of indoles are included in the region R2, the control unit 64 determines that the stress level of the subject is high as the concentration of phenols is relatively high in the ratio of the concentrations of phenols and indoles. When the determined detection value of the concentration of phenols and the determined detection value of the concentration of indoles are included in the region R1, the control unit 64 determines that the stress level of the subject is sufficiently low. The control unit 64 may convert the stress level of the subject into a score that is a numerical value of 0 to 100, for example. The control unit 64 may transmit the measured stress level of the subject to the electronic device 3 via the communication unit 62.

<Individual Difference Correction Process>

The following individual difference correction process may be performed when a certain period of time has elapsed since the start of use of the stress measurement system 1, during maintenance, or the like.

The reference value $PO_0$ is a typical value determined when the stress measurement system 1 is designed. For this reason, it may be necessary to adjust stress measurement of the control unit 64 depending on individual differences for the subject, in particular, depending on the proportion of bacteria present in the intestine. For example, if the concentration of phenols is relatively high due to individual differences for the subject, the subject may be determined not to feel stress even if the stress level of the subject becomes high. The individual difference correction process can be performed to perform stress measurement appropriate for each subject. The proportion of bacteria present in the intestine of the subject may be the proportions of the numbers of bacteria for specific species of bacteria present in the intestine of the subject or the ratio of the proportions. The specific species of bacteria in the intestine may include at least one of bifidobacteria, lactobacilli, corynebacteria, staphylococci, Walsh bacteria (clostridia), Bacteroides, Escherichia coli, Enterobacter, Pseudomonas, or Candida. The number of bacteria for a specific species of bacteria in the intestine of the subject may be measured by examination of the feces of the subject by an inspection institute or the like.

When performing the stress measurement process, the control unit 64 stores the detection value of the concentration of phenols and the detection value of the concentration of indoles in the storage unit 61. The storage unit 61 accumulates the detection value of the concentration of phenols and the detection value of the concentration of indoles together with, for example, date and time information. For example, when a certain period of time has elapsed since the start of use of the stress measurement system 1, the control unit 64 determines a new reference value $PO_1$ on the basis of a plurality of previous detection values stored in the storage unit 61. For example, the control unit 64 may perform statistical processing of previous detection values of the concentration of phenols and previous detection values of the concentration of indoles to determine a new reference value $PO_1$. In the statistical processing, for example, the control unit 64 may determine an average value or a median value. The original reference value $PO_0$ is replaced with a reference value $PO_1$ that takes individual differences for each subject into account. As illustrated in Fig. 7, the region R1 and the region R2 move in accordance with the change from the original reference value $PO_0$ to the new reference value $PO_1$.

[Example Operation of Stress Measurement System]

<Operation in Detection of Type and Concentration of Gas>

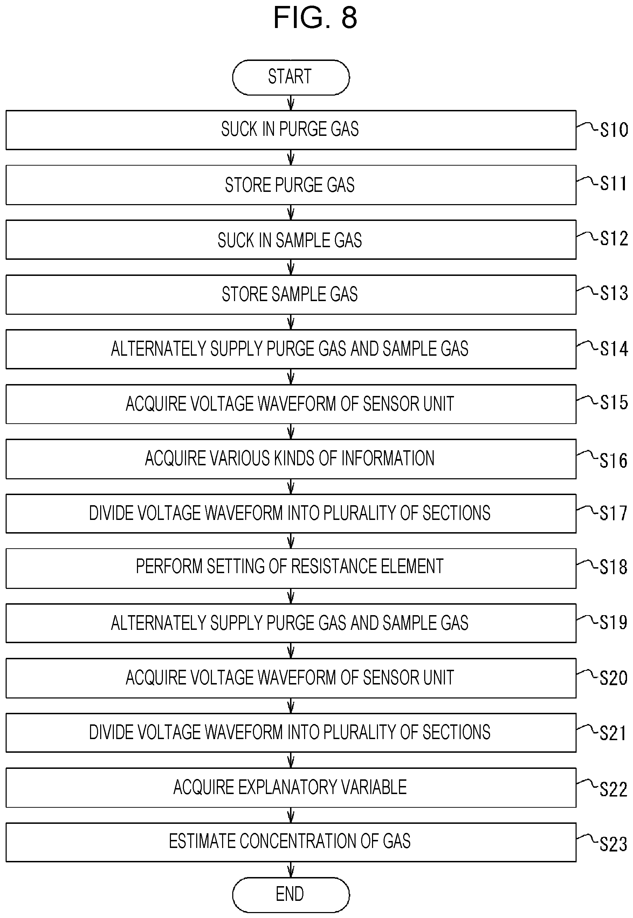

Fig. 8 is a flowchart illustrating an example operation of the stress measurement system 1 in detection of the type and concentration of a gas. The control unit 64 may start the process illustrated in Fig. 8 after a predetermined time has elapsed since it was detected that the subject rose from the toilet seat 2B on the basis of the detection result of the sensor unit 63.

The control unit 64 causes the purge gas to be sucked in through the second suction hole 21 (step S10). The control unit 64 causes the purge gas to be sucked in through the second suction hole 21 to store the purge gas in the second reservoir 41 (step S11).

The control unit 64 causes the sample gas to be sucked in through the first suction hole 20 after a predetermined time has elapsed since it was detected that the subject sat on the toilet seat 2B on the basis of the detection result of the sensor unit 63 (step S12). The control unit 64 causes the sample gas to be sucked in through the first suction hole 20 to store the sample gas in the first reservoir 40 (step S13).

The control unit 64 controls the second supply unit 51 and the first supply unit 50 to alternately supply the purge gas and the sample gas to the chamber 30 (step S14). The control unit 64 acquires a voltage waveform output by the sensor unit 31 in the chamber 30 (step S15).

The control unit 64 acquires, for example, various kinds of information from the outside via the storage unit 61 or the communication unit 62 (step S16). The various kinds of information include the information related to the prediction equation described above, and the like.

The control unit 64 divides the voltage waveform output by the sensor unit 31 into a plurality of sections by, for example, dividing the voltage waveform by a predetermined interval along the time axis (step S17).

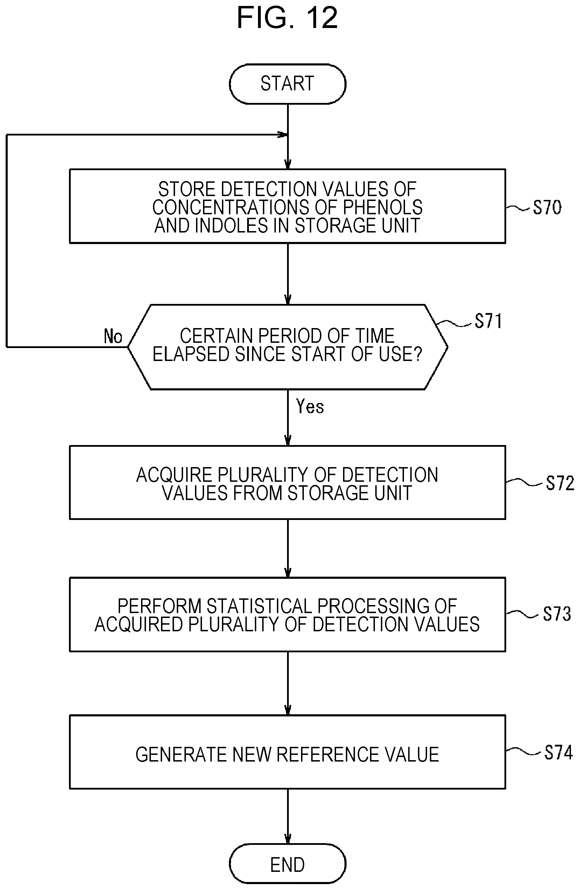

The control unit 64 performs setting of the resistance element 31R to adjust the resolution of the sensor unit 31 (step S18). The details of the processing of step S18 will be described below with reference to Fig. 12.

The control unit 64 executes the processing of steps S19 and S20 in a way similar to that of the processing of steps S14 and S15.

The control unit 64 executes the processing of step S21 in a way similar to that of the processing of step S17.

The control unit 64 acquires, based on information on computation for acquiring an explanatory variable, which is included in the information related to the prediction equation acquired in the processing of step S16, an explanatory variable used in the prediction equation (step S22) .

The control unit 64 substitutes the explanatory variable acquired in the processing of step S22 into the prediction equation to detect the concentration of a predetermined gas (step S23). For example, the control unit 64 substitutes the explanatory variables x11, x22, and x33 into prediction equation (1) described above to detect the concentration y1 of the predetermined gas.

The control unit 64 executes the process illustrated in Fig. 8 for each different prediction equation. The process illustrated in Fig. 8 can be executed for each different prediction equation to detect the type and concentration of a gas.

In the processing of step S11, the control unit 64 may determine whether the cleanliness of the purge gas is high. Further, if the cleanliness of the purge gas is high, the control unit 64 may store the purge gas in the second reservoir 41. In this case, the control unit 64 may control the second supply unit 51 to supply the purge gas to the chamber 30. Further, the control unit 64 may determine, based on the detection result of the sensor unit 31, whether the cleanliness of the purge gas is high. When the stress measurement system 1 includes a dedicated sensor unit that detects the cleanliness of the purge gas, the control unit 64 may determine whether the cleanliness of the purge gas is high on the basis of the detection result of the dedicated sensor unit.

<Operation in Determination of Prediction Equation>

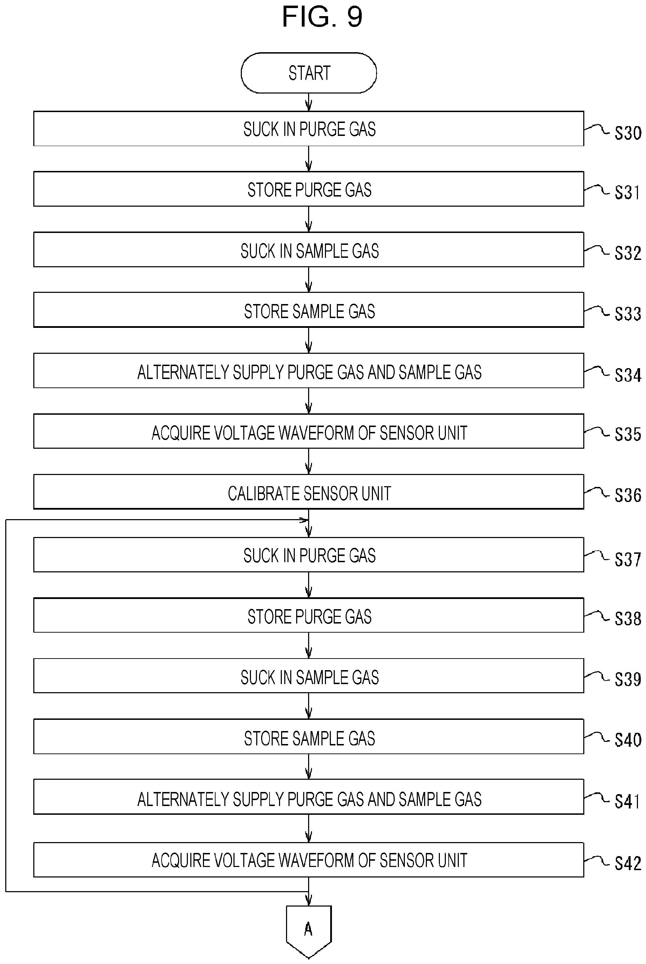

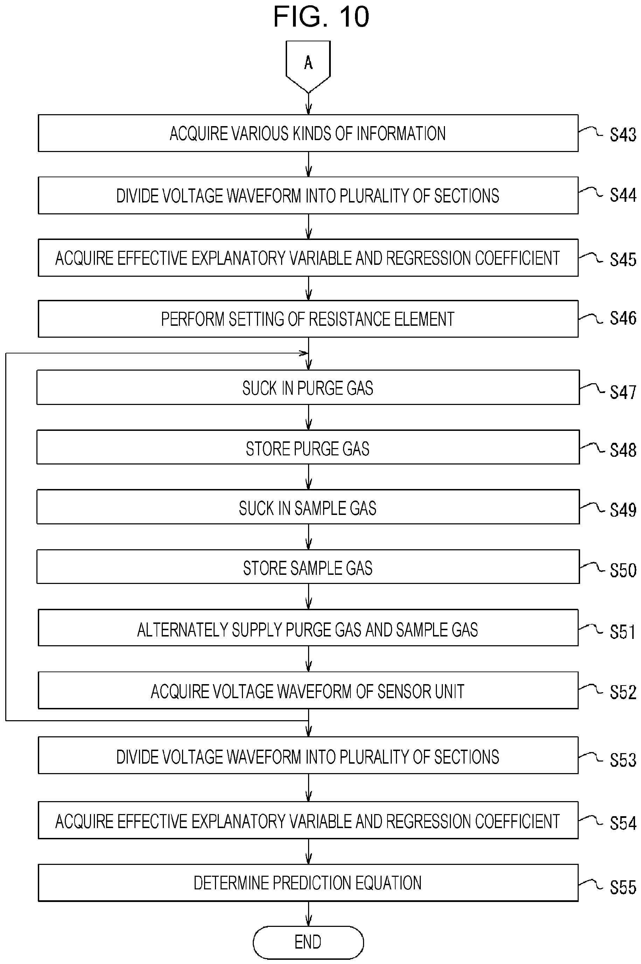

Fig. 9 and Fig. 10 are a flowchart illustrating the operation of the stress measurement system 1 illustrated in Fig. 1 in determination of a prediction equation. The process illustrated in Fig. 9 and Fig. 10 may be executed before the stress measurement system 1 is shipped as a product. The control unit 64 may start the process illustrated in Fig. 9 in accordance with a program incorporated therein in advance or when a control signal for providing an instruction to suck in the purge gas is received from the outside via the communication unit 62. Here, when a prediction equation is to be determined, a plurality of standard gases whose concentrations are maximized may be used as the sample gas.

The control unit 64 executes the processing of steps S30 and 31 in a way similar to that of the processing of steps S10 and S11 illustrated in Fig. 8.

The control unit 64 executes the processing of steps S32 and 33 in a way similar to that of the processing of steps S12 and 13 illustrated in Fig. 8 when a control signal for providing an instruction to suck in the sample gas is received from the outside via the communication unit 62.

The control unit 64 executes the processing of steps S34 and S35 in a way similar to that of the processing of steps S14 and S15 illustrated in Fig. 8. As described above, since a standard gas whose concentration is maximized is used, the amplitude of the voltage waveform of the sensor unit 31 acquired in the processing of step S35 can be maximized.

The control unit 64 calibrates the sensor unit 31 (step S36) on the basis of the voltage waveform of the sensor unit 31 acquired in the processing of step S35. As described above, the amplitude of the voltage waveform of the sensor unit 31 acquired in the processing of step S35 can be maximized. This enables the sensor unit 31 to be more accurately calibrated in the processing of step S36.

The control unit 64 executes the processing of steps S37 to S42 in a way similar to that of the processing of steps S30 to S35. As described above, when a prediction equation is to be determined, a plurality of standard gases are used as the sample gas. Thus, the control unit 64 repeatedly executes the processing of steps S37 to S42 a number of times corresponding to the number of standard gases used.

The control unit 64 proceeds to the process illustrated in Fig. 10. The control unit 64 acquires various kinds of information from the outside via the storage unit 61 or the communication unit 62 (step S43). The various kinds of information include a model equation in the multiple regression analysis (for example, model equation (2) described above), information related to the standard gases, and the like.

The control unit 64 executes the processing of step S44 in a way similar to that of the processing of step S17 illustrated in Fig. 8.

For example, the control unit 64 executes machine learning with training data on the voltage waveform to acquire an effective explanatory variable and a regression coefficient in the model equation (for example, model equation (2) described above) (step S45).

The control unit 64 performs setting of the resistance element 31R to adjust the resolution of the sensor unit 31 (step S46). The details of the processing of step S46 will be described below with reference to Fig. 12.

The control unit 64 executes the processing of steps S47 to S52 in a way similar to that of the processing of steps S37 to S42 illustrated in Fig. 9. The control unit 64 repeatedly executes the processing of steps S47 to S52, in a way similar to that of the processing of steps S37 to S42 illustrated in Fig. 9, a number of times corresponding to the number of standard gases used to determine the prediction equation. The control unit 64 executes the processing of steps S53 and S54 in a way similar to that of the processing of steps S44 and S45.

The control unit 64 determines a prediction equation for detecting the concentration of the gas n (for example, prediction equation (1) described above) (step S55).

The control unit 64 does not need to execute the processing of steps S30 to S36 illustrated in Fig. 9. When the control unit 64 executes the processing of steps S30 to S36 illustrated in Fig. 9, a plurality of standard gases whose concentrations are maximized may be used only for the processing of steps S30 to S36.

In some cases, the effective explanatory variable and regression coefficient acquired in the processing of step S54 may be different from the effective explanatory variable and regression coefficient acquired in the processing of step S45. In this case, the control unit 64 may execute the processing of steps S30 to S42 illustrated in Fig. 9 again and then execute the processing of steps S44 and S45 again.

<Operation in Measurement of Stress>

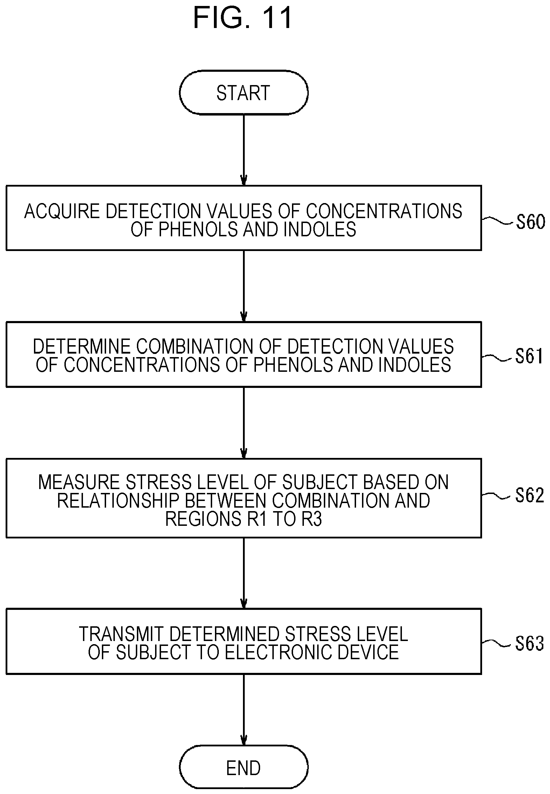

Fig. 11 is a flowchart illustrating an example operation of the stress measurement system 1 in measurement of stress, that is, a stress measurement method.

The control unit 64 acquires detection values of the concentrations of phenols and indoles (step S60). The control unit 64 determines a combination of the detection values of the concentrations of phenols and indoles (step S61). The control unit 64 measures the stress level of the subject on the basis of the relationship between the determined combination and the regions R1 to R3 (step S62). The data or relational expression for defining the regions R1 to R3 may be stored in the storage unit 61. The control unit 64 transmits the determined stress level of the subject to the electronic device 3 via the communication unit 62 (step S63).

<Operation in Correction of Individual Differences>

Fig. 12 is a flowchart illustrating the operation of the stress measurement system in correction of individual differences.

When measuring stress, the control unit 64 stores the detection values of the concentrations of phenols and indoles in the storage unit 61 (step S70). If the certain period of time has not elapsed since the start of use (No in step S71), the control unit 64 returns to the processing of step S70. If the certain period of time has elapsed since the start of use (Yes in step S71), the control unit 64 acquires a plurality of detection values from the storage unit 61 (step S72). The control unit 64 performs statistical processing of the acquired plurality of detection values (step S73). The control unit 64 generates, based on the result of the statistical processing in step S73, the reference value $PO_1$ that takes individual differences for each subject into account (step S74). The control unit 64 may update, based on the difference between the new reference value $PO_1$ and the original reference value $POo$, the data or relational expression for defining the regions R1 to R3, which is stored in the storage unit 61.

In the stress measurement system 1 according to this embodiment, as described above, the sensor unit 31 detects a plurality of detection target gases based on substances contained in a specimen of a subject, and outputs a plurality of detection values corresponding to the respective detection results of the plurality of detection target gases. Then, the control unit 64 determines the stress level of the subject on the basis of the combination of the plurality of detection values. The stress measurement system 1 according to this embodiment measures stress by using a plurality of detection target gases from the specimen of the subject, which eliminates the time and effort that the subject spends, such as wearing a measurement device or drawing blood. According to this embodiment, therefore, it is possible to provide the stress measurement system 1 capable of measuring the stress level of a subject without taking time and effort.

The drawings describing embodiments according to the present disclosure are schematic. Dimensions, ratios, and the like in the drawings do not necessarily match the actual ones.

While an embodiment according to the present disclosure has been described with reference to the drawings and examples, it should be noted that various modifications or changes can be easily made by a person skilled in the art on the basis of the present disclosure. Accordingly, it should be noted that these modifications or changes fall within the scope of the present disclosure. For example, the functions and the like included in each component or the like can be rearranged in any manner that is not logically contradictory, and a plurality of components can be combined into one or divided.

For example, the adsorbent 40b and the adsorbent 40c may be separately heated and desorbed such that phenols and indoles are concentrated as separate gases and can be separately fed into the chamber 30. That is, a phenols-concentrated gas and an indoles-concentrated gas can be measured in separate steps to estimate the concentration of phenols in the phenols-concentrated gas measurement step and estimate the concentration of indoles in the indoles-concentrated gas measurement step. The method described above exhibits an effect of improving the accuracy of measurement when the sensor unit 31 does not include a gas sensor with a high gas sensor sensitivity of phenols to indoles or a gas sensor with a high gas sensor sensitivity of indoles to phenols.

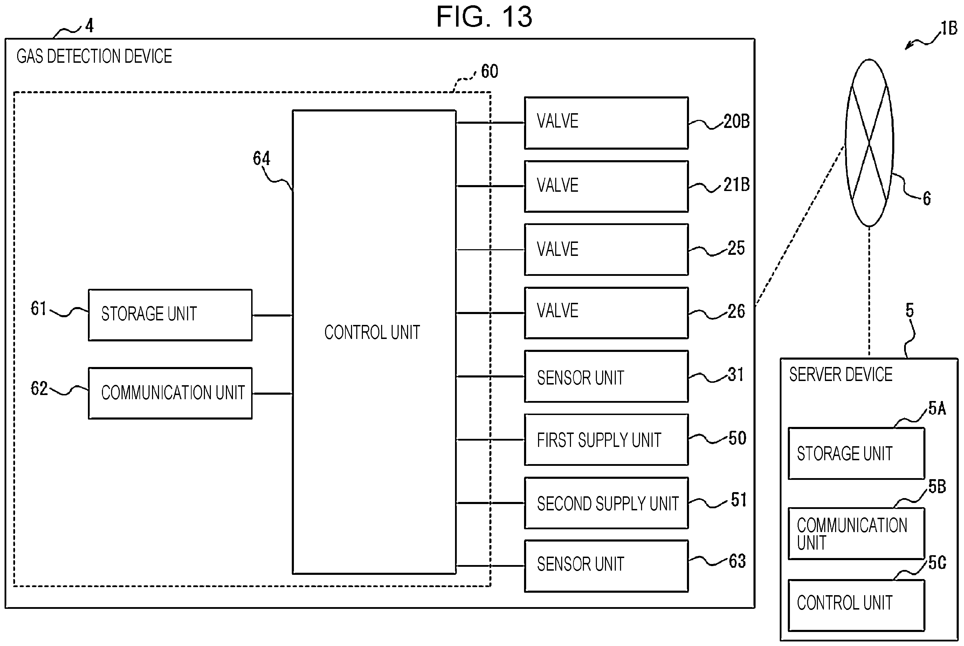

For example, in the embodiment described above, as illustrated in Fig. 3, the stress measurement system 1 has been described as a single device. However, a stress measurement system of the present disclosure is not limited to the single device and may include a plurality of independent devices. A stress measurement system of the present disclosure may have a configuration as illustrated in Fig. 13, for example.

A stress measurement system 1B illustrated in Fig. 13 includes a stress measurement device 4 and a server device 5. The stress measurement device 4 and the server device 5 are capable of communicating with each other via a network 6. A portion of the network 6 may be wired or wireless. The stress measurement device 4 has a configuration similar to the configuration of the stress measurement system 1 illustrated in Fig. 2 and Fig. 3. The server device 5 includes a storage unit 5A, a communication unit 5B, and a control unit 5C. The control unit 5C is capable of executing the processes of the control unit 64 illustrated in Fig. 3 described above. For example, the control unit 5C can acquire a voltage waveform output by the sensor unit 31 illustrated in Fig. 2 via the communication unit 5B and the network 6. Further, the control unit 5C can detect the type and concentration of a gas contained in the sample gas on the basis of multiple regression analysis using characteristics of the voltage waveform as explanatory variables.

For example, in a period from voltage measurement of a gas by the sensor unit 31 to the subsequent suction period, the purge gas may be introduced into the first reservoir 40, the second reservoir 42, or the sensor unit 31. This period may include a period during which a heater of at least any one of the first reservoir 40 and the second reservoir 41 is heated. This configuration allows the first reservoir 40 and the adsorbents 40a, 40b, and 40c to be refreshed, and allows the second reservoir 41 and the adsorbents 41a, 41b, and 41c to be refreshed.

In the present disclosure, descriptions such as "first" and "second" are identifiers for distinguishing the respective configurations. The configurations distinguished by the descriptions such as "first" and "second" in the present disclosure may be interchangeably numbered. For example, a first suction hole and a second suction hole may exchange their identifiers "first" and "second". The identifiers are exchanged simultaneously. Even after the identifiers are exchanged, the respective configurations are distinguishable. The identifiers may be deleted. Configurations without identifiers are distinguished using reference numerals. Only the description of identifiers such as "first" and "second" in the present disclosure should not be used as a basis for interpreting the order of the configurations or for determining the presence of identifiers with smaller numbers.

A network, as used herein, includes, unless otherwise specified, the Internet, an ad hoc network, a LAN (Local Area Network), a WAN (Wide Area Network), a MAN (Metropolitan Area Network), a cellular network, a WWAN (Wireless Wide Area Network), a WPAN (Wireless Personal Area Network), a PSTN (Public Switched Telephone Network), a terrestrial wireless network or other networks, or any combination thereof. Components of a wireless network include, for example, access points (for example, Wi-Fi access points), femtocells, and the like. Further, a wireless communication device can connect to a wireless network that uses Wi-Fi, Bluetooth, cellular communication technology (for example, CDMA (Code Division Multiple Access), TDMA (Time Division Multiple Access), FDMA (Frequency Division Multiple Access), OFDMA (Orthogonal Frequency Division Multiple Access), SC-FDMA (Single-Carrier Frequency Division Multiple Access), or other wireless technology and/or technology standard. The network can employ one or more technologies, and such technologies include, for example, UTMS (Universal Mobile Telecommunications System), LTE (Long Term Evolution), EV-DO (Evolution-Data Optimized or Evolution-Data Only), GSM (Global System for Mobile communications), WiMAX (Worldwide Interoperability for Microwave Access), CDMA-2000 (Code Division Multiple Access-2000), or TD-SCDMA (Time Division Synchronous Code Division Multiple Access).

Further, it should be noted that a system including various modules and/or units that implement specific functions is disclosed herein and that these modules and units are illustrated schematically to provide a brief description of their functionality and are not necessarily indicative of specific hardware and/or software. In this sense, these modules, units, and other components may be hardware and/or software implemented to substantially execute the specific functions described herein. Various functions of different components may be implemented using any combination or separation of hardware and/or software and may be used individually or in any combination. In addition, an input/output, or I/O, device or a user interface including but not limited to a keyboard, a display, a touch screen, a pointing device, etc. can be connected to the system either directly or with intervention of an I/O controller. Accordingly, various aspects of the present disclosure can be embodied in many different forms. All such embodiments fall within the scope of the present disclosure.

Reference Signs List

| 1, 1B | stress measurement system |
|---|---|
| 2 | toilet |
| 2A | toilet bowl |
| 2B | toilet seat |
| 3 | electronic device |
| 3A | display unit |
| 4 | stress measurement device |
| 5 | server device |
| 5A | storage unit |
| 5B | communication unit |
| 5C | control unit |
| 6 | network |
| 10 | housing |
| 20 | first suction hole |
| 21 | second suction hole |
| 20A, 21A | air blower |
| 20B, 21B | valve |
| 22 | discharge path |
| 23, 23-1, 23-2, 24, 24-1, 24-2, 27, 27-1, 27-2, 27-3, 28 | flow path |
| 25, 26 | valve |
| 30 | chamber |
| 31, 31-1, 31-2, 31-3 | sensor unit |

| 31S | sensor element |
| 31R | resistance element |
| 40 | first reservoir |
| 41 | second reservoir |
| 40a, 40b, 40c, 41a, 41b, 41c | adsorbent |
| 50 | first supply unit |
| 51 | second supply unit |
| 52 | third supply unit |
| 60 | circuit board |
| 61 | storage unit |
| 62 | communication unit |
| 63 | sensor unit |
| 64 | control unit |
| P1 | power supply terminal |
| P2 | ground terminal |

## Claims

1. A stress measurement system comprising:

   a sensor unit that detects a plurality of detection target gases based on substances contained in a specimen of a subject and outputs a plurality of detection values corresponding to respective detection results of the plurality of detection target gases; and
   a control unit that determines a stress level of the subject, based on a combination of the plurality of detection values.

2. The stress measurement system according to Claim 1, wherein the substances contained in the specimen include a substance serving as a raw material for a brain neurotransmitter.

3. The stress measurement system according to Claim 1 or 2, wherein the substances contained in the specimen include phenols and indoles, and
   wherein the control unit determines the stress level of the subject, based on a combination of a detection value of a detection target gas based on the phenols and a detection value of a detection target gas based on the indoles.

4. The stress measurement system according to Claim 3, wherein the phenols comprise phenol or cresol.

5. The stress measurement system according to Claim 3 or 4, wherein the indoles comprise indole or skatole.

6. The stress measurement system according to any one of Claims 1 to 5, wherein the sensor unit outputs the plurality of detection values in accordance with respective concentrations of the plurality of detection target gases.

7. The stress measurement system according to any one of Claims 1 to 6, further comprising a storage unit that stores the plurality of detection values,
   wherein the control unit adjusts the stress level to be determined, based on the plurality of detection values stored in the storage unit, the plurality of detection values being previous values.

8. The stress measurement system according to any one of Claims 1 to 7, further comprising a reservoir in which the plurality of detection target gases to be supplied to the sensor unit are concentrated.

9. The stress measurement system according to Claim 8, wherein the reservoir includes an adsorbent, and the plurality of detection target gases are concentrated by selective adsorption using the adsorbent and heating and desorption using the adsorbent.

10. The stress measurement system according to any one of Claims 1 to 8, wherein the control unit determines the stress level of the subject by using a reference value.

11. The stress measurement system according to Claim 10, wherein the control unit corrects the reference value in accordance with an individual difference for the subject.

12. The stress measurement system according to Claim 11, wherein the control unit performs statistical processing of previous detection values of the subject to correct the reference value.

13. The stress measurement system according to any one of Claims 1 to 12, wherein the sensor unit is arranged in a chamber, and
wherein the control unit alternately supplies a purge gas and a sample gas to the chamber.

14. The stress measurement system according to Claim 13, wherein the sensor unit comprises a plurality of sensor units, and
wherein each of the plurality of sensor units is located in a corresponding one of a plurality of chambers into which the chamber is divided.

15. A stress measurement method comprising:

detecting a plurality of detection target gases from a specimen of a subject and respective concentrations of the plurality of detection target gases; and
determining a stress level of the subject, based on a combination of the concentrations of the plurality of detection target gases.

# FIG. 1

# FIG. 2

# FIG. 3

# FIG. 4

# FIG. 5

# FIG. 6

DETECTION VALUE [V] OF
CONCENTRATION OF PHENOLS

R3

$PO_0$

INCREASE
IN STRESS

R1          R2

DETECTION VALUE [V] OF
CONCENTRATION OF INDOLES

# FIG. 7

DETECTION VALUE [V] OF
CONCENTRATION OF PHENOLS

R3

$PO_1$

R1          R2

DETECTION VALUE [V] OF
CONCENTRATION OF INDOLES

# FIG. 8

START

| SUCK IN PURGE GAS | S10 |

| STORE PURGE GAS | S11 |

| SUCK IN SAMPLE GAS | S12 |

| STORE SAMPLE GAS | S13 |

| ALTERNATELY SUPPLY PURGE GAS AND SAMPLE GAS | S14 |

| ACQUIRE VOLTAGE WAVEFORM OF SENSOR UNIT | S15 |

| ACQUIRE VARIOUS KINDS OF INFORMATION | S16 |

| DIVIDE VOLTAGE WAVEFORM INTO PLURALITY OF SECTIONS | S17 |

| PERFORM SETTING OF RESISTANCE ELEMENT | S18 |

| ALTERNATELY SUPPLY PURGE GAS AND SAMPLE GAS | S19 |

| ACQUIRE VOLTAGE WAVEFORM OF SENSOR UNIT | S20 |

| DIVIDE VOLTAGE WAVEFORM INTO PLURALITY OF SECTIONS | S21 |

| ACQUIRE EXPLANATORY VARIABLE | S22 |

| ESTIMATE CONCENTRATION OF GAS | S23 |

END

# FIG. 9

START

| SUCK IN PURGE GAS | S30 |

| STORE PURGE GAS | S31 |

| SUCK IN SAMPLE GAS | S32 |

| STORE SAMPLE GAS | S33 |

| ALTERNATELY SUPPLY PURGE GAS AND SAMPLE GAS | S34 |

| ACQUIRE VOLTAGE WAVEFORM OF SENSOR UNIT | S35 |

| CALIBRATE SENSOR UNIT | S36 |

| SUCK IN PURGE GAS | S37 |

| STORE PURGE GAS | S38 |

| SUCK IN SAMPLE GAS | S39 |

| STORE SAMPLE GAS | S40 |

| ALTERNATELY SUPPLY PURGE GAS AND SAMPLE GAS | S41 |

| ACQUIRE VOLTAGE WAVEFORM OF SENSOR UNIT | S42 |

A

# FIG. 10

A

| | |
|---|---|
| ACQUIRE VARIOUS KINDS OF INFORMATION | S43 |
| DIVIDE VOLTAGE WAVEFORM INTO PLURALITY OF SECTIONS | S44 |
| ACQUIRE EFFECTIVE EXPLANATORY VARIABLE AND REGRESSION COEFFICIENT | S45 |
| PERFORM SETTING OF RESISTANCE ELEMENT | S46 |
| SUCK IN PURGE GAS | S47 |
| STORE PURGE GAS | S48 |
| SUCK IN SAMPLE GAS | S49 |
| STORE SAMPLE GAS | S50 |
| ALTERNATELY SUPPLY PURGE GAS AND SAMPLE GAS | S51 |
| ACQUIRE VOLTAGE WAVEFORM OF SENSOR UNIT | S52 |
| DIVIDE VOLTAGE WAVEFORM INTO PLURALITY OF SECTIONS | S53 |
| ACQUIRE EFFECTIVE EXPLANATORY VARIABLE AND REGRESSION COEFFICIENT | S54 |
| DETERMINE PREDICTION EQUATION | S55 |

END

# FIG. 11

```
        START

ACQUIRE DETECTION VALUES OF CONCENTRATIONS     S60
        OF PHENOLS AND INDOLES

DETERMINE COMBINATION OF DETECTION VALUES      S61
   OF CONCENTRATIONS OF PHENOLS AND INDOLES

MEASURE STRESS LEVEL OF SUBJECT BASED ON       S62
  RELATIONSHIP BETWEEN COMBINATION AND
          REGIONS R1 TO R3

TRANSMIT DETERMINED STRESS LEVEL               S63
   OF SUBJECT TO ELECTRONIC DEVICE

          END
```

# FIG. 12

START

STORE DETECTION VALUES OF
CONCENTRATIONS OF PHENOLS
AND INDOLES IN STORAGE UNIT ~S70

CERTAIN PERIOD OF TIME
ELAPSED SINCE START OF USE? ~S71

No

Yes

ACQUIRE PLURALITY OF DETECTION
VALUES FROM STORAGE UNIT ~S72

PERFORM STATISTICAL PROCESSING OF
ACQUIRED PLURALITY OF DETECTION VALUES ~S73

GENERATE NEW REFERENCE VALUE ~S74

END

FIG. 13

GAS DETECTION DEVICE — 4

1B

6

60

64

CONTROL UNIT

VALVE — 20B

VALVE — 21B

VALVE — 25

VALVE — 26

SENSOR UNIT — 31

FIRST SUPPLY UNIT — 50

SECOND SUPPLY UNIT — 51

SENSOR UNIT — 63

61 — STORAGE UNIT

62 — COMMUNICATION UNIT

SERVER DEVICE — 5

STORAGE UNIT — 5A

COMMUNICATION UNIT — 5B

CONTROL UNIT — 5C

EP 4 067 903 A1

**INTERNATIONAL SEARCH REPORT**

| International application No. |
| --- |
| PCT/JP2020/043062 |

**A. CLASSIFICATION OF SUBJECT MATTER**
G01N 33/497(2006.01)i; G01N 33/50(2006.01)i
FI: G01N33/50 Z; G01N33/497 D

According to International Patent Classification (IPC) or to both national classification and IPC

**B. FIELDS SEARCHED**

Minimum documentation searched (classification system followed by classification symbols)
G01N33/497; G01N33/50

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | WO 2019/198648 A1 (SHISEIDO CO., LTD.) 17 October 2019 (2019-10-17) claims, paragraphs [0017]-[0091] | 1, 6-7, 10, 15<br>8-14<br>2-5 |
| Y | WO 2014/192674 A1 (NTT DOCOMO, INC.) 04 December 2014 (2014-12-04) claims | 8-14 |
| A | JP 4-204056 A (HITACHI, LTD.) 24 July 1992 (1992-07-24) entire text all drawings | 1-15 |
| A | WO 2019/008974 A1 (PANASONIC IP MANAGEMENT CO., LTD.) 10 January 2019 (2019-01-10) entire text all drawings | 1-15 |
| A | JP 2007-101344 A (KAO CORP.) 19 April 2007 (2007-04-19) entire text all drawings | 1-15 |
| A | JP 9-43182 A (HITACHI, LTD.) 14 February 1997 (1997-02-14) entire text all drawings | 1-15 |

☒ Further documents are listed in the continuation of Box C.          ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 21 December 2020 (21.12.2020) | 12 January 2021 (12.01.2021) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2020/043062

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2019-148514 A (RICOH CO., LTD.) 05 September 2019 (2019-09-05) entire text all drawings | 1-15 |
| A | US 2011/0035158 A1 (BANOS, Peter Theophilos) 10 February 2011 (2011-02-10) entire text all drawings | 1-15 |
| A | SAKUMA, Kenji et al., "Analysis of Odor Compounds in Feces of Mice that Were Exposed to Various Stresses during Breeding", Experimental Animals, 2013, 62(2), 101-107 entire text all drawings | 1-15 |

Form PCT/ISA/210 (continuation of second sheet) (January 2015)

**INTERNATIONAL SEARCH REPORT**

Information on patent family members

| International application No. |
|---|
| PCT/JP2020/043062 |

| Patent Documents referred in the Report | Publication Date | Patent Family | Publication Date |
|---|---|---|---|
| WO 2019/198648 A1 | 17 Oct. 2019 | TW 201947223 A | |
| WO 2014/192674 A1 | 04 Dec. 2014 | US 2016/0120458 A1 claims<br>EP 3006915 A | |
| JP 4-204056 A | 24 Jul. 1992 | (Family: none) | |
| WO 2019/008974 A1 | 10 Jan. 2019 | US 2020/0008729 A1 entire text all drawings<br>CN 110546504 A | |
| JP 2007-101344 A | 19 Apr. 2007 | (Family: none) | |
| JP 9-43182 A | 14 Feb. 1997 | (Family: none) | |
| JP 2019-148514 A | 05 Sep .2019 | (Family: none) | |
| US 2011/0035158 A1 | 10 Feb. 2011 | WO 2011/017616 A1 | |

Form PCT/ISA/210 (patent family annex) (January 2015)

**EP 4 067 903 A1**

**Patent documents cited in the description**

- JP 2019213551 A

- JP 2010273700 A